# EUROPEAN PATENT APPLICATION

(11) **EP 4 414 708 A1**
(43) Date of publication of application: **14.08.2024**
(21) Application number: 22878704.0
(22) Date of filing: 11.08.2022
(51) Int. Cl.: G01N 33/574, G01N 33/82, G01N 33/92, G01N 33/68, G01N 30/72

(54) **BIOMARKER FOR CANCER DIAGNOSIS AND USE THEREOF**

(30) Priority: 06.10.2021 KR 20210132381; 08.04.2022 KR 20220043775
(71) Applicant: Innobation Bio Co., Ltd., Seoul 03929 (KR)
(72) Inventor: KIM, Seung ku, Yongin-si,Gyeonggi-do 16865 (KR); KIM, Ki Tae, Seoul 06148 (KR)
(74) Representative: J A Kemp LLP
(86) International application number: PCT/KR2022/011990
(87) International publication number: WO 2023/058883

(57) **Abstract**

The present invention relates to biomarkers for cancer diagnosis and uses thereof, and more particularly to a composition for diagnosing or predicting cancer prognosis, comprising an agent for measuring levels of at least one biomarker selected from the group consisting of Complement Component 7 (C7), Dodecanoyl-L-carnitine (DC), Lysophosphatidylcholine (LPC), Histidine-Rich Glycoprotein (HRG) and Osteopontin in blood, and a method of using the biomarker to provide information about the diagnosis or prognosis of cancer.

In the present invention, it was confirmed that the level of complement component 7 (C7), dodecanoyl-L-carnitine (DC), lysophosphatidylcholine (LPC), histidine-rich glycoprotein (HRG), and/or osteopontin (OPN) in blood of lung and/or hepatic cancer patients has been found to have different patterns than that of normal individuals, and the combination of these markers has been shown to improve the diagnosis of various cancers, including lung or hepatic cancer. Accordingly, the biomarkers of the present invention can be useful in cancer diagnosis and prognosis.

## Description

### [Technical field]

The present invention relates to biomarkers for cancer diagnosis and uses thereof, and more particularly to a composition for diagnosing or predicting cancer prognosis, comprising an agent for measuring levels of at least one biomarker selected from the group consisting of Complement Component 7 (C7), Dodecanoyl-L-carnitine (DC), Lysophosphatidylcholine (LPC), Histidine-Rich Glycoprotein (HRG) and Osteopontin in blood, and a method of using the biomarker to provide information about the diagnosis or prognosis of cancer.

### [Background art]

Cancer is a disease in which cells multiply indefinitely and interfere with normal cellular functions, most commonly liver, lung, stomach, breast, colon, and ovarian cancers, but can occur in virtually any tissue.

Initially, cancer diagnosis was based on external changes in biological tissues caused by the growth of cancer cells, but in recent years, diagnosis has been attempted by detecting trace amounts of biomolecules such as blood, glyco chains, and DNA in the tissues or cells of living organisms. However, the most commonly used cancer diagnosis methods are tissue samples obtained through biopsy or imaging.

Among them, biopsy can be painful for the patient, expensive, and can take a long time to diagnose. In addition, if the patient has actual cancer, there is a risk that the biopsy process may cause the cancer to metastasize, and if the biopsy cannot obtain a tissue sample, the disease cannot be diagnosed until the suspect tissue is surgically removed.

In particular, hepatic cancer, or hepatocellular carcinoma, is the most common type of hepatic cancer in adults and accounts for the third leading cause of death from cancer (Stefaniuk P, et al., World J Gastroenterol., 16:418-424, 2010). The most commonly used tumor biomarker for hepatic cancer is alpha-Fetoprotein (AFP), which is only present in extremely low concentrations in adults (7-10 ng/mL or less for normal), so an increase in AFP levels in blood can indicate a significant increase in tumor cells. Generally, hepatic cancer is diagnosed where AFP is elevated above or equal to 20 ng/mL, but diagnosis using AFP has a low sensitivity of about 40%. This means that AFP-negative hepatic cancers account for more than 40% of all hepatic cancers, so there is a need for additional markers that can effectively increase the diagnosis rate of hepatic cancer with greater accuracy.

Other serum markers that are not established diagnostic criteria but may be helpful in the diagnosis of hepatic cancer include descarboxyprothrombin (DCP), Prothrombin Induced by Vitamin K Absence II (PIVKA-II), glycosylated AFP to total AFP (L3 fraction) distribution, alpha fucosidase (AFU), glypican 3, and HSP-70. However, each of them is mostly meaningful as a prognostic factor and is not yet used as a screening test due to its low accuracy when used alone, and to solve this problem, research is being conducted on various markers that can accurately diagnose hepatic cancer (Korean Laid-open Patent No. 10-2014-0115490; Korean Laid-open Patent No. 10-2016-0072027).

In addition, lung cancer is one of the most lethal cancers in the world, and the current diagnosis of lung cancer relies heavily on imaging methods (X-ray, CT, MRI, etc.). However, more than half of lung cancer patients are already inoperable at the time of diagnosis, and even if surgery is performed, many of them cannot be completely resected. Therefore, early diagnosis and treatment of lung cancer is the most important to increase the cure rate of lung cancer, but lung cancer is difficult to diagnose in detail because there are limited useful markers for diagnosis. Therefore, there is a need to develop cancer-specific markers in biological samples and methods to diagnose cancer with high accuracy and precision using these markers.

### [Disclosure]

### [Technical Problem]

Accordingly, in a diligent effort to identify markers that can more accurately diagnose cancer, the present inventors surprisingly found that the levels of Complement Component 7 (C7), Dodecanoyl-L-carnitine (DC), Lysophosphatidylcholine (LPC), and Histidine-Rich Glycoprotein (HRG) and Osteopontin (OPN) in blood of patients with lung or hepatic cancer have different patterns from those of normal people, and confirmed that the combination of these markers improved the diagnostic ability of various cancers, including lung and hepatic cancer, and completed the invention.

Accordingly, it is an object of the present invention to provide a composition for diagnosing cancer comprising an agent for measuring the level of one or more markers selected from the group consisting of Complement Component 7 (C7), Dodecanoyl-L-carnitine (DC), Lysophosphatidylcholine (LPC), Histidine-Rich Glycoprotein (HRG) and Osteopontin (OPN) in blood, plasma or serum, a kit for diagnosing cancer using the composition, and a method for providing information for diagnosing cancer.

Another object of the present invention is to provide a composition for predicting cancer prognosis comprising an agent for measuring the level of one or more markers selected from the group consisting of Complement Component 7 (C7), Dodecanoyl-L-carnitine (DC), Lysophosphatidylcholine (LPC), Histidine-Rich Glycoprotein (HRG) and Osteopontin (OPN) in blood, plasma or serum, a kit for predicting cancer prognosis using the composition, and a method for providing information for predicting cancer prognosis.

### [Technical Solution]

To accomplish the above purpose, the present invention provides a biomarker composition for diagnosing cancer, wherein the composition comprises one or more biomarker(s) selected from the group consisting of Complement Component 7 (C7), Dodecanoyl-L-carnitine (DC), Lysophosphatidylcholine (LPC), Histidine-Rich Glycoprotein (HRG), and Osteopontin (OPN), and wherein the biomarker is derived from blood.

In one preferred embodiment of the present invention, the blood may be whole blood, plasma, or serum.

In another preferred embodiment of the present invention, the cancer may be lung cancer, pancreatic cancer, biliary tract cancer, colon cancer, breast cancer, gastric cancer, brain tumor, kidney cancer, hepatic cancer, or cervical cancer.

In another preferred embodiment of the present invention, the lysophosphatidylcholine (LPC) may be lysophosphatidylcholine 16:0 (LPC16) or lysophosphatidylcholine 18:0 (LPC18).

In another preferred embodiment of the present invention, where the concentration of C7 biomarker in blood is higher than that of a normal control;
where the concentration of OPN biomarker in blood is higher than that of a normal control;
where the concentration of DC biomarker in blood is lower than that of a normal control;
where the concentration of LPC biomarker in blood is lower than that of a normal control; or
where the concentration of Histidine-Rich Glycoprotein (HRG) biomarker in blood is lower than that of a normal control group, it can be diagnosed or determined to have cancer.

In another preferred embodiment of the present invention, where the cut-off value of C7 biomarker is 230 to 240 µg/ml, and the concentration of C7 in blood is above or equal to the cut-off value;
where the cutoff value for DC biomarker is 14 to 17 ng/mL, and the concentration of DC in blood is below or equal to the cutoff value;
where the cutoff value for HRG biomarker is 90 to 95 µg/ml, and a concentration of HRG in blood below or equal to the cutoff value; or
where the cutoff value for LPC biomarker is 40 to 45 µg/ml, and a concentration of LPC in blood below the cut-off value, it can be diagnosed or determined to have lung cancer.

In another preferred embodiment of the present invention, where the cut-off value of C7 biomarker is 245 to 270 µg/ml, and the concentration of C7 biomarker in blood is above or equal to the cut-off value;
where the cutoff value for DC biomarker is 12 to 15 ng/ml, and the concentration of DC biomarker in blood is below or equal to the cutoff value;
where the cutoff value for HRG biomarker is 85 to 90 µg/ml, and the concentration of HRG biomarker in blood is below or equal to the cutoff value;
where the cutoff value for OPN biomarker is 80 to 90 ng/mL, and the concentration of OPN biomarker in blood above or equal to the cutoff value, or
where the cut-off value of LPC biomarker is 44 to 50 µg/ml, and the concentration of LPC biomarker in blood below the cut-off value, it can be diagnosed or determined to have hepatic cancer.

In another preferred embodiment of the present invention,
(1) where the biomarkers are C7 and DC, the cancer can be diagnosed by the ratio of C7 concentration : DC concentration (C7/DC);
(2) where the biomarkers are C7 and LPC, the cancer can be diagnosed by the ratio of C7 concentration : LPC concentration (C7/LPC);
(3) where the biomarkers are C7 and HRG, the cancer can be diagnosed by the ratio of C7 concentration : HRG concentration (C7/HRG);
(4) where the biomarkers are C7, DC, and LPC, the cancer can be diagnosed by the ratio of C7 concentration : (sum of DC concentration and LPC concentration) (C7/(DC + LPC));
(5) where the biomarkers are C7, DC, and HRG, the cancer is diagnosed by the ratio of C7 concentration : (sum of DC concentration and HRG concentration) (C7/(DC + HRG));
(6) where the biomarkers are C7, DC, HRG, and LPC, the cancer can be diagnosed by the ratio of C7 concentration : (sum of DC concentration, HRG concentration and LPC concentration) (C7/(DC + HRG + LPC));
(7) where the biomarkers are C7, HRG, and LPC, the cancer can be diagnosed by the ratio of C7 concentration : (sum of HRG concentration and LPC concentration) (C7/(HRG + LPC));
(8) where the biomarkers are OPN and DC, the cancer can be diagnosed by the ratio of OPN concentration : DC concentration (OPN/DC);
(9) where the biomarkers are OPN and LPC, the cancer can be diagnosed by the ratio of OPN concentration : LPC concentration (OPN/LPC);
(10) where the biomarkers are OPN and HRG, the cancer can be diagnosed by the ratio of OPN concentration : HRG concentration (OPN/HRG);
(11) where the biomarkers are OPN, DC, and LPC, the cancer can be diagnosed by the ratio of OPN concentration : (sum of DC concentration and LPC concentration) (OPN/(DC + LPC)); or
(12) where the biomarkers are OPN, DC, and HRG, the cancer can be diagnosed by the ratio of OPN concentration : (sum of DC concentration and HRG concentration) (OPN/(DC + HRG)).

In another preferred embodiment of the present invention,
(1) where the cut-off value for the ratio of C7 concentration : DC concentration (C7/DC) is 10 to 12 and the ratio of C7 concentration : DC concentration in blood is above or equal to the cut-off value, it can be diagnosed or determined to have lung cancer;
(2) where the cut-off value for the ratio of C7 concentration : LPC concentration (C7/LPC) is 3.5 to 5 and the ratio of C7 concentration : LPC concentration in blood is above or equal to the cut-off value, it can be diagnosed or determined to have lung cancer;
(3) where the cut-off value for the ratio of C7 concentration : HRG concentration (C7/HRG) is 2 to 2.5 and the ratio of C7 concentration : HRG concentration in blood is above or equal to the cut-off value, it can be diagnosed or determined to have lung cancer;
(4) where the cut-off value for the ratio of C7 concentration : (sum of DC concentration and LPC concentration) (C7/(DC + LPC)) is 2.5 ~ 3.0 and the ratio of C7 concentration : (sum of DC concentration and LPC concentration) in blood is above or equal to the cut-off value, it can be diagnosed or determined to have lung cancer;
(5) where the cut-off value for the ratio of C7 concentration : (sum of DC concentration and HRG concentration) (C7/(DC + HRG)) is 1.5 to 2.0 and the ratio of C7 concentration : (sum of DC concentration and HRG concentration) in blood is above or equal to the cut-off value, it can be diagnosed or determined to have lung cancer;
(6) where the cut-off value for the ratio of C7 concentration : (sum of DC concentration, HRG concentration and LPC concentration) (C7/(DC + HRG + LPC)) is 1 to 1.5 and the ratio of C7 concentration : (sum of DC concentration, HRG concentration and LPC concentration) in blood, plasma or serum is above or equal to the cut-off value, it can be diagnosed or determined to have lung cancer;
(7) where the cut-off value for the ratio of C7 concentration : (sum of HRG concentration and LPC concentration) (C7/(HRG + LPC)) is 1.2 ~ 2 and the ratio of C7 concentration : (sum of HRG concentration and LPC concentration) in blood is above or equal to the cut-off value, it can be diagnosed or determined to have lung cancer;
(8) where the cut-off value for the ratio of OPN concentration : DC concentration (OPN/DC) is 3 to 5 and the ratio of OPN concentration : DC concentration in blood is above or equal to the cutoff value, it can be diagnosed or determined to have lung cancer;
(9) where the cut-off value for the ratio of OPN concentration : LPC concentration (OPN/LPC) is 1.5 to 2.0 and the ratio of OPN concentration : LPC concentration in blood is above or equal to the cut-off value, it can be diagnosed or determined to have lung cancer;
(10) where the cut-off value for the ratio of OPN concentration : HRG concentration (OPN/HRG) is 0.8 to 1.2 and the ratio of OPN concentration : HRG concentration in blood is above or equal to the cut-off value, it can be diagnosed or determined to have lung cancer;
(11) where the cut-off value for the ratio of OPN concentration : (sum of DC concentration and LPC concentration) (OPN/(DC + LPC)) is 0.9 to 1.5 and the ratio of OPN concentration : (sum of DC concentration and LPC concentration) in blood is above or equal to the cut-off value, it can be diagnosed or determined to have lung cancer; or
(12) where the cut-off value for the ratio of OPN concentration : (sum of DC concentration and HRG concentration) (OPN/(DC + HRG)) is 0.5 to 1.2 and the ratio of OPN concentration : (sum of DC concentration and HRG concentration) in blood is above or equal to the cut-off value, it can be diagnosed or determined to have lung cancer.

In another preferred embodiment of the present invention,
(1) where the cut-off value for the ratio of C7 concentration : DC concentration (C7/DC) is 8 to 12 and the ratio of C7 concentration: DC concentration ratio in blood is above or equal to the cutoff value, it can be diagnosed or determined to have hepatic cancer;
(2) where the cut-off value for the ratio of C7 concentration : LPC concentration ratio (C7/LPC) is 3.5 to 5 and the ratio of C7 concentration : LPC concentration in blood is above or equal to the cut-off value, it can be diagnosed or determined to have hepatic cancer;
(3) where the cut-off value for the ratio of C7 concentration : HRG concentration (C7/HRG) is 2 to 2.5 and the ratio of C7 concentration : HRG concentration in blood is above or equal to the cut-off value, it can be diagnosed or determined to have hepatic cancer;
(4) where the cut-off value for the ratio of C7 concentration : (sum of DC concentration and LPC concentration) (C7/(DC + LPC)) is 2 to 4 and the ratio of C7 concentration : (sum of DC concentration and LPC concentration) in blood is above or equal to the cut-off value, it can be diagnosed or determined to have hepatic cancer;
(5) where the cut-off value for the ratio of C7 concentration : (sum of DC concentration and HRG concentration) (C7/(DC + HRG)) is 1.5 ~ 2 and the ratio of C7 concentration : (sum of DC concentration and HRG concentration) in blood is above or equal to the cut-off value, it can be diagnosed or determined to have hepatic cancer;
(6) where the cut-off value for the ratio of C7 concentration : (sum of DC concentration, HRG concentration, and LPC concentration) (C7/(DC + HRG + LPC)) is 1.2 to 2 and the ratio of C7 concentration : (sum of DC concentration, HRG concentration, and LPC concentration) in blood is above or equal to the cut-off value, it can be diagnosed or determined to have hepatic cancer;
(7) where the cut-off value for the ratio of C7 concentration : (sum of HRG concentration and LPC concentration) (C7/(HRG + LPC)) is 1 to 3 and the ratio of C7 concentration : (sum of HRG concentration and LPC concentration) in blood is above or equal to the cut-off value, it can be diagnosed or determined to have hepatic cancer;
(8) where the cut-off value for the ratio of OPN concentration : DC concentration (OPN/DC) is 3 to 5 and the ratio of OPN concentration: DC concentration in blood is above or equal to the cutoff value, it can be diagnosed or determined to have hepatic cancer;
(9) where the cut-off value for the ratio of OPN concentration : LPC concentration (OPN/LPC) is 1.5 to 2.5 and the ratio of OPN concentration : LPC concentration in blood is above or equal to the cut-off value, it can be diagnosed or determined to have hepatic cancer;
(10) where the cut-off value for the ratio of OPN to HRG (OPN/HRG) is 3 to 5 and the ratio of OPN concentration : HRG concentration in blood is above or equal to the cut-off value, it can be diagnosed or determined to have hepatic cancer;
(11) where the cut-off value for the ratio of OPN concentration : (sum of DC concentration and LPC concentration) (OPN/(DC + LPC)) is 0.8 ~ 2 and the ratio of OPN concentration : (sum of DC concentration and LPC concentration) in blood is above or equal to the cut-off value, it can be diagnosed or determined to have hepatic cancer; or
(12) where the cut-off value for the ratio of OPN concentration : (sum of DC concentration and HRG concentration) (OPN/(DC + HRG)) is 0.5 to 1.2 and the ratio of OPN concentration : (sum of DC concentration and HRG concentration) in blood is above or equal to the cut-off value, it can be diagnosed or determined to have hepatic cancer.

The present invention also provides a composition for diagnosing cancer, comprising an agent for measuring levels in blood of the biomarker composition for diagnosing cancer.

In a preferred embodiment of the present invention, an agent for measuring the level of a C7, HRG, or OPN biomarker is an agent for measuring protein levels and may comprise an antibody, interacting protein, ligand, nanoparticle, or aptamer that specifically binds to a protein or peptide fragment of each marker.

In another preferred embodiment of the present invention, an agent for measuring levels of DC biomarker or LPC biomarker may be an agent or a preparation for mass spectrometry.

The present invention also provides a kit for diagnosing cancer, comprising an agent for measuring levels in blood of the biomarker composition for diagnosing cancer.

In one preferred embodiment of the present invention, the kit may be an enzyme linked immunosorbent assay (ELISA) kit, a protein chip kit, a rapid kit, or a multiple reaction monitoring (MRM) kit.

The present invention further provides a method of providing information for diagnosing cancer, comprising (a) measuring levels of one or more biomarkers selected from the group consisting of Complement Component 7 (C7), Dodecanoyl-L-carnitine (DC), Lysophosphatidylcholine (LPC), Histidine-Rich Glycoprotein (HRG), and Osteopontin (OPN) in the patient's blood; and (b) comparing levels of the biomarker to those from a normal control sample.

In a preferred embodiment of the present invention, the cancer may be lung cancer, pancreatic cancer, biliary tract cancer, colon cancer, breast cancer, gastric cancer, brain tumor, kidney cancer, hepatic cancer, or cervical cancer.

In another preferred embodiment of the present invention, the blood in step (a) may be whole blood, plasma, or serum.

In another preferred embodiment of the present invention, the lysophosphatidylcholine (LPC) may be lysophosphatidylcholine 16:0 (LPC16) or lysophosphatidylcholine 18:0 (LPC18).

In another preferred embodiment of the present invention, the method of providing information for diagnosing cancer may further comprise the step of providing information that cancer is present,
where the concentration of C7 biomarker in blood is higher than that of a normal control;
where the concentration of OPN biomarker in blood is higher than that of a normal control;
where the concentration of DC biomarker in blood is lower than that of a normal control;
where the concentration of LPC biomarker in blood is lower than that of a normal control; or
where the concentration of Histidine-Rich Glycoprotein (HRG) biomarker in blood is lower than that of a normal control group.

In another preferred embodiment of the present invention, the method of providing information for diagnosing cancer may provide information that lung cancer is present,
where the cut-off value of C7 biomarker is 230 to 240 µg/ml, and the concentration of C7 in blood is above or equal to the cut-off value;
where the cutoff value for DC biomarker is 14 to 17 ng/mL, and the concentration of DC in blood is below or equal to the cutoff value;
where the cutoff value for HRG biomarker is 90 to 95 µg/ml, and a concentration of HRG in blood below or equal to the cutoff value; or
where the cutoff value for LPC biomarker is 40 to 45 µg/ml, and a concentration of LPC in blood below the cut-off value.

In another preferred embodiment of the present invention, the method of providing information for diagnosing cancer may provide information that hepatic cancer is present,
where the cut-off value of C7 biomarker is 245 to 270 µg/ml, and the concentration of C7 biomarker in blood is above or equal to the cut-off value;
where the cutoff value for DC biomarker is 12 to 15 ng/ml, and the concentration of DC biomarker in blood is below or equal to the cutoff value;
where the cutoff value for HRG biomarker is 85 to 90 µg/ml, and the concentration of HRG biomarker in blood is below or equal to the cutoff value;
where the cutoff value for OPN biomarker is 80 to 90 ng/mL, and the concentration of OPN biomarker in blood above or equal to the cutoff value, or
where the cut-off value of LPC biomarker is 44 to 50 µg/ml, and the concentration of LPC biomarker in blood below the cut-off value.

In another preferred embodiment of the present invention,
(1) where the biomarkers are C7 and DC, the information for diagnosing cancer is measured or provided by the ratio of C7 concentration : DC concentration (C7/DC);
(2) where the biomarkers are C7 and LPC, the information for diagnosing cancer is measured or provided by the ratio of C7 concentration : LPC concentration (C7/LPC);
(3) where the biomarkers are C7 and HRG, the information for diagnosing cancer is measured or provided by the ratio of C7 concentration : HRG concentration (C7/HRG);
(4) where the biomarkers are C7, DC, and LPC, the information for diagnosing cancer is measured or provided by the ratio of C7 concentration : (sum of DC concentration and LPC concentration) (C7/(DC + LPC));
(5) where the biomarkers are C7, DC, and HRG, the information for diagnosing cancer is measured or provided by the ratio of C7 concentration : (sum of DC concentration and HRG concentration) (C7/(DC + HRG));
(6) where the biomarkers are C7, DC, HRG, and LPC, the information for diagnosing cancer is measured or provided by the ratio of C7 concentration : (sum of DC concentration, HRG concentration and LPC concentration) (C7/(DC + HRG + LPC));
(7) where the biomarkers are C7, HRG, and LPC, the information for diagnosing cancer is measured or provided by the ratio of C7 concentration : (sum of HRG concentration and LPC concentration) (C7/(HRG + LPC));
(8) where the biomarkers are OPN and DC, the information for diagnosing cancer is measured or provided by the ratio of OPN concentration : DC concentration (OPN/DC);
(9) where the biomarkers are OPN and LPC, the information for diagnosing cancer is measured or provided by the ratio of OPN concentration : LPC concentration (OPN/LPC);
(10) where the biomarkers are OPN and HRG, the information for diagnosing cancer is measured or provided by the ratio of OPN concentration : HRG concentration (OPN/HRG);
(11) where the biomarkers are OPN, DC, and LPC, the information for diagnosing cancer is measured or provided by the ratio of OPN concentration : (sum of DC concentration and LPC concentration) (OPN/(DC + LPC)); or
(12) where the biomarkers are OPN, DC, and HRG, the information for diagnosing cancer is measured or provided by the ratio of OPN concentration : (sum of DC concentration and HRG concentration) (OPN/(DC + HRG)).

In another preferred embodiment of the present invention,
(1) where the cut-off value for the ratio of C7 concentration : DC concentration (C7/DC) is 10 to 12 and the ratio of C7 concentration : DC concentration in blood is above or equal to the cut-off value, the cancer is lung cancer;
(2) where the cut-off value for the ratio of C7 concentration : LPC concentration (C7/LPC) is 3.5 to 5 and the ratio of C7 concentration : LPC concentration in blood is above or equal to the cut-off value, the cancer is lung cancer;
(3) where the cut-off value for the ratio of C7 concentration : HRG concentration (C7/HRG) is 2 to 2.5 and the ratio of C7 concentration : HRG concentration in blood is above or equal to the cut-off value, the cancer is lung cancer;
(4) where the cut-off value for the ratio of C7 concentration : (sum of DC concentration and LPC concentration) (C7/(DC + LPC)) is 2.5 ~ 3.0 and the ratio of C7 concentration : (sum of DC concentration and LPC concentration) in blood is above or equal to the cut-off value, the cancer is lung cancer;
(5) where the cut-off value for the ratio of C7 concentration : (sum of DC concentration and HRG concentration) (C7/(DC + HRG)) is 1.5 to 2.0 and the ratio of C7 concentration : (sum of DC concentration and HRG concentration) in blood is above or equal to the cut-off value, the cancer is lung cancer;
(6) where the cut-off value for the ratio of C7 concentration : (sum of DC concentration, HRG concentration and LPC concentration) (C7/(DC + HRG + LPC)) is 1 to 1.5 and the ratio of C7 concentration : (sum of DC concentration, HRG concentration and LPC concentration) in blood, plasma or serum is above or equal to the cut-off value, the cancer is lung cancer;
(7) where the cut-off value for the ratio of C7 concentration : (sum of HRG concentration and LPC concentration) (C7/(HRG + LPC)) is 1.2 ~ 2 and the ratio of C7 concentration : (sum of HRG concentration and LPC concentration) in blood is above or equal to the cut-off value, the cancer is lung cancer;
(8) where the cut-off value for the ratio of OPN concentration : DC concentration (OPN/DC) is 3 to 5 and the ratio of OPN concentration : DC concentration in blood is above or equal to the cutoff value, the cancer is lung cancer;
(9) where the cut-off value for the ratio of OPN concentration : LPC concentration (OPN/LPC) is 1.5 to 2.0 and the ratio of OPN concentration : LPC concentration in blood is above or equal to the cut-off value, the cancer is lung cancer;
(10) where the cut-off value for the ratio of OPN concentration : HRG concentration (OPN/HRG) is 0.8 to 1.2 and the ratio of OPN concentration : HRG concentration in blood is above or equal to the cut-off value, the cancer is lung cancer;
(11) where the cut-off value for the ratio of OPN concentration : (sum of DC concentration and LPC concentration) (OPN/(DC + LPC)) is 0.9 to 1.5 and the ratio of OPN concentration : (sum of DC concentration and LPC concentration) in blood is above or equal to the cut-off value, the cancer is lung cancer; or
(12) where the cut-off value for the ratio of OPN concentration : (sum of DC concentration and HRG concentration) (OPN/(DC + HRG)) is 0.5 to 1.2 and the ratio of OPN concentration : (sum of DC concentration and HRG concentration) in blood is above or equal to the cut-off value, the cancer is lung cancer.

In another preferred embodiment of the present invention,
(1) where the cut-off value for the ratio of C7 concentration : DC concentration (C7/DC) is 8 to 12 and the ratio of C7 concentration: DC concentration ratio in blood is above or equal to the cutoff value, the cancer is hepatic cancer;
(2) where the cut-off value for the ratio of C7 concentration : LPC concentration ratio (C7/LPC) is 3.5 to 5 and the ratio of C7 concentration : LPC concentration in blood is above or equal to the cut-off value, the cancer is hepatic cancer;
(3) where the cut-off value for the ratio of C7 concentration : HRG concentration (C7/HRG) is 2 to 2.5 and the ratio of C7 concentration : HRG concentration in blood is above or equal to the cut-off value, the cancer is hepatic cancer;
(4) where the cut-off value for the ratio of C7 concentration : (sum of DC concentration and LPC concentration) (C7/(DC + LPC)) is 2 to 4 and the ratio of C7 concentration : (sum of DC concentration and LPC concentration) in blood is above or equal to the cut-off value, the cancer is hepatic cancer;
(5) where the cut-off value for the ratio of C7 concentration : (sum of DC concentration and HRG concentration) (C7/(DC + HRG)) is 1.5 ~ 2 and the ratio of C7 concentration : (sum of DC concentration and HRG concentration) in blood is above or equal to the cut-off value, the cancer is hepatic cancer;
(6) where the cut-off value for the ratio of C7 concentration : (sum of DC concentration, HRG concentration, and LPC concentration) (C7/(DC + HRG + LPC)) is 1.2 to 2 and the ratio of C7 concentration : (sum of DC concentration, HRG concentration, and LPC concentration) in blood is above or equal to the cut-off value, the cancer is hepatic cancer;
(7) where the cut-off value for the ratio of C7 concentration : (sum of HRG concentration and LPC concentration) (C7/(HRG + LPC)) is 1 to 3 and the ratio of C7 concentration : (sum of HRG concentration and LPC concentration) in blood is above or equal to the cut-off value, the cancer is hepatic cancer;
(8) where the cut-off value for the ratio of OPN concentration : DC concentration (OPN/DC) is 3 to 5 and the ratio of OPN concentration: DC concentration in blood is above or equal to the cutoff value, the cancer is hepatic cancer;
(9) where the cut-off value for the ratio of OPN concentration : LPC concentration (OPN/LPC) is 1.5 to 2.5 and the ratio of OPN concentration : LPC concentration in blood is above or equal to the cut-off value, the cancer is hepatic cancer;
(10) where the cut-off value for the ratio of OPN to HRG (OPN/HRG) is 3 to 5 and the ratio of OPN concentration : HRG concentration in blood is above or equal to the cut-off value, the cancer is hepatic cancer;
(11) where the cut-off value for the ratio of OPN concentration : (sum of DC concentration and LPC concentration) (OPN/(DC + LPC)) is 0.8 ~ 2 and the ratio of OPN concentration : (sum of DC concentration and LPC concentration) in blood is above or equal to the cut-off value, the cancer is hepatic cancer; or
(12) where the cut-off value for the ratio of OPN concentration : (sum of DC concentration and HRG concentration) (OPN/(DC + HRG)) is 0.5 to 1.2 and the ratio of OPN concentration : (sum of DC concentration and HRG concentration) in blood is above or equal to the cut-off value, the cancer is hepatic cancer.

In another preferred embodiment of the present invention, the measuring levels of one or more biomarkers selected from the group consisting of C7, HRG and OPN in step (a) above can be performed using protein chip analysis, immunometry, ligand binding assay, matrix desorption/ionization time of flight mass spectrometry (MALDI-TOF) analysis, surface enhanced laser desorption/ionization time of flight mass spectrometry (SELDI-TOF) analysis, radioimmunoassay, radioimmunodiffusion, or ouchterlony immunodiffusion, rocket immunoelectrophoresis, tissue immunostaining, complement fixation assay, two-dimensional electrophoresis analysis, liquid chromatography-mass spectrometry (LC-MS), liquid chromatography-mass spectrometry/mass spectrometry (LC-MS/MS), western blot, and/or enzyme linked immunosorbent assay (ELISA).

In another preferred embodiment of the present invention, the measuring levels of one or more biomarkers selected from the group consisting of DC and LPC in step (a) above can be performed using liquid chromatography-mass spectrometry (LC-MS).

To accomplish other purposes,
The present invention also provides a composition for predicting cancer prognosis, wherein the composition comprises at least one biomarker selected from the group consisting of Complement Component 7 (C7), Dodecanoyl-L-carnitine (DC), Lysophosphatidylcholine (LPC), Histidine-Rich Glycoprotein (HRG), and Osteopontin (OPN), and wherein the biomarker is derived from blood

The present invention also provides a composition for predicting cancer prognosis comprising an agent that measures the level of the biomarker for predicting cancer prognosis.

The present invention also provides a kit for diagnosing cancer prognosis comprising an agent that measures the level of the biomarker for predicting cancer prognosis.

The present invention also provides a method of providing information for predicting cancer prognosis, comprising the step of measuring a level of the biomarker for predicting cancer prognosis.

### [Advantageous Effects]

In the present invention, it was confirmed that the level of complement component 7 (C7), dodecanoyl-L-carnitine (DC), lysophosphatidylcholine (LPC), histidine-rich glycoprotein (HRG), and/or osteopontin (OPN) in blood of lung and/or hepatic cancer patients has been found to have different patterns than that of normal individuals, and the combination of these markers has been shown to improve the diagnosis of various cancers, including lung or hepatic cancer. Accordingly, the biomarkers of the present invention can be useful in cancer diagnosis and prognosis.

### [Description of Drawings]

FIG. 1(A) shows data of C7 concentration in blood obtained from lung cancer patients (LC) and normal controls (Nor). In the figure, the values are expressed as mean (mean) ± standard error of the mean (SEM), and N indicates the number of patients.
Figure 1(B) shows data of DC concentration in blood obtained from lung cancer patients and healthy controls.
Figure 1(C) shows data of HRG concentration in blood obtained from lung cancer patients and healthy controls.
Figure 1(D) shows data of LPC concentration in blood obtained from lung cancer patients and healthy controls.
FIG. 2(A) shows data of C7 and DC concentration in blood obtained from lung cancer patients and healthy controls, expressed as C7/DC concentration ratio.
FIG. 2(B) shows data of C7 concentration and LPC concentration in blood obtained from lung cancer patients and normal controls, expressed as C7/LPC concentration ratio.
FIG. 2(C) shows the data of C7 concentration and HRG concentration in blood obtained from lung cancer patients and normal controls, expressed as C7/HRG concentration ratio.
FIG. 2(D) shows data of C7 concentration, DC concentration, and LPC concentration in blood obtained from lung cancer patients and healthy controls, expressed as C7/(DC + LPC) concentration ratio.
FIG. 2(E) shows data of C7 concentration, DC concentration, and HRG concentration in blood obtained from lung cancer patients and healthy controls, expressed as C7/(DC + HRG) concentration ratio.
FIG. 2(F) shows data of C7 concentration, DC concentration, HRG concentration, and LPC concentration in blood obtained from lung cancer patients and healthy controls, expressed as C7/(DC + HRG + LPC) concentration ratio.
FIG. 3(A) shows data of C7 concentration, HRG concentration, and LPC concentration in blood obtained from lung cancer patients and healthy controls, expressed as C7/(HRG + LPC) concentration ratio.
FIG. 3(B) shows data of OPN concentration and DC concentration in blood obtained from lung cancer patients and healthy controls, expressed as OPN/DC concentration ratio.
FIG. 3(C) shows data of OPN and LPC concentrations in blood from lung cancer patients and healthy controls, expressed as OPN/LPC concentration ratio.
FIG. 3(D) shows data of OPN and HRG concentrations in blood obtained from lung cancer patients and healthy controls, expressed as OPN/HRG concentration ratio.
FIG. 3(E) shows data of OPN concentration, DC concentration, and LPC concentration in blood obtained from lung cancer patients and healthy controls, expressed as OPN/(DC + LPC) concentration ratio.
Figure 3(F) shows data of OPN concentration, DC concentration, and HRG concentration in blood obtained from lung cancer patients and healthy controls, expressed as OPN/(DC + HRG) concentration ratio.
Figure 4(A) shows data of C7 concentration in blood obtained from a group of hepatic cancer patients (HC) and a normal control (Nor).
Figure 4(B) shows data of DC concentration in blood obtained from hepatic cancer patients and healthy controls.
Figure 4(C) shows data of HRG concentration in blood obtained from hepatic cancer patients and normal controls.
Figure 4(D) shows data of OPN concentration in blood obtained from hepatic cancer patients and normal controls.
FIG. 4(E) shows data of LPC concentration in blood obtained from hepatic cancer patients and normal controls.
FIG. 5(A) shows data of C7 concentration and DC concentration in blood obtained from hepatic cancer patients and normal controls, expressed as C7/DC concentration ratio.
FIG. 5(B) shows data of C7 concentration and LPC concentration in blood obtained from hepatic cancer patients and normal controls, expressed as C7/LPC concentration ratio.
FIG. 5(C) shows data of C7 concentration and HRG concentration in blood obtained from hepatic cancer patients and normal controls, expressed as C7/HRG concentration ratio.
FIG. 5(D) shows data of C7 concentration, DC concentration, and LPC concentration in blood obtained from hepatic cancer patients and normal controls, expressed as C7/(DC + LPC) concentration ratio.
FIG. 5(E) shows data of C7 concentration, DC concentration, and HRG concentration in blood obtained from hepatic cancer patients and normal controls, expressed as C7/(DC + HRG) concentration ratio.
FIG. 5(F) shows data of C7 concentration, DC concentration, HRG concentration, and LPC concentration in blood obtained from hepatic cancer patients and normal controls, expressed as C7/(DC + HRG + LPC) concentration ratio.
FIG. 6(A) shows data of C7 concentration, HRG concentration, and LPC concentration in blood obtained from hepatic cancer patients and normal controls, expressed as C7/(HRG + LPC) concentration ratio.
FIG. 6(B) shows data of OPN and DC concentrations in blood obtained from a hepatic cancer patient and a healthy control, represented as OPN/DC concentration ratio
FIG. 6(C) shows data of OPN and LPC concentrations in blood obtained from hepatic cancer patients and normal controls, expressed as OPN/LPC concentration ratio
FIG. 6(D) shows data of OPN concentrations and HRG concentrations in blood obtained from hepatic cancer patients and normal controls, expressed as OPN/HRG concentration ratio.
FIG. 6(E) shows data of OPN concentration, DC concentration, and LPC concentration in blood obtained from hepatic cancer patients and normal controls, expressed as OPN/(DC + LPC) concentration ratio.
FIG. 6(F) shows data of OPN concentration, DC concentration, and HRG concentration in blood obtained from hepatic cancer patients and normal controls, expressed as OPN/(DC + HRG) concentration ratio.

### [Mode of the Invention]

The present invention will now be described in detail.

### Biomarker Composition for diagnosing cancer

In one aspect, the invention relates to a biomarker composition for diagnosing cancer, wherein the composition comprises at least one biomarker selected from the group consisting of Complement Component 7 (C7), Dodecanoyl-L-carnitine (DC), Lysophosphatidylcholine (LPC), Histidine-Rich Glycoprotein (HRG), and Osteopontin (OPN), and wherein the biomarker is derived from blood.

In the present invention, the blood may be whole blood, plasma or serum.

In the present invention, the cancer may be lung cancer, pancreatic cancer, biliary tract cancer, colon cancer, breast cancer, gastric cancer, brain tumor, kidney cancer, hepatic cancer, or cervical cancer, and preferably hepatic cancer or lung cancer.

In the present invention, the lysophosphatidylcholine (LPC) may be lysophosphatidylcholine 16:0 (LPC16) or lysophosphatidylcholine 18:0 (LPC18).

In the present invention, the present composition can diagnose cancer using one marker selected from the group consisting of C7, DC, LPC, HRG and OPN, preferably by combining 2 to 4 markers, more preferably 'combination of C7 and DC markers', 'combination of C7 and LPC markers', 'combination of C7 and HRG markers', 'combination of C7, DC and LPC markers', 'combination of C7, DC and HRG markers', 'combination of C7, DC, HRG and LPC markers', 'combination of C7, HRG and LPC markers', 'combination of OPN and DC markers', 'combination of OPN and LPC markers', 'combination of OPN and HRG markers', 'combination of OPN, DC and LPC markers', or 'combination of OPN, DC and HRG markers'.

In a specific embodiment of the present invention, blood from a normal person, a patient with lung cancer (LC), and a patient with hepatic cancer (HC) was obtained, and the concentrations of C7, DC, LPC, HRG, and OPN in blood were measured.

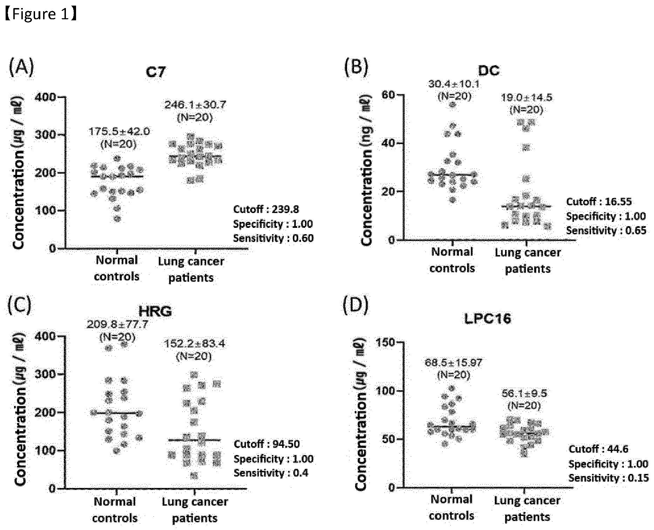

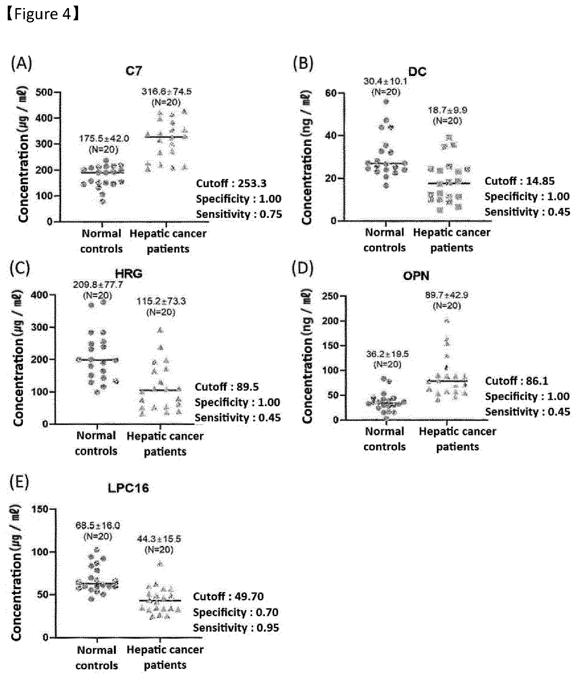

As a result, we found that patients with lung cancer or hepatic cancer had different concentrations of markers in their blood than healthy individuals, as shown in FIGS. 1(A) to 1(D) (lung cancer) and FIGS. 4(A) to 4(E) (hepatic cancer).

Specifically, where the concentration of C7 biomarker in blood is higher than that of a normal control;
where the concentration of OPN biomarker in blood is higher than that of a normal control;
where the concentration of DC biomarker in blood is lower than that of a normal control;
where the concentration of LPC biomarker in blood is lower than that of a normal control; or
where the concentration of Histidine-Rich Glycoprotein (HRG) biomarker in blood is lower than that of a normal control group, it can be diagnosed or determined to have cancer.

More specifically, where the cut-off value of C7 biomarker is 230 to 240 µg/ml, and the concentration of C7 in blood is above or equal to the cut-off value;
where the cutoff value for DC biomarker is 14 to 17 ng/mL, and the concentration of DC in blood is below or equal to the cutoff value;
where the cutoff value for HRG biomarker is 90 to 95 µg/ml, and a concentration of HRG in blood below or equal to the cutoff value; or
where the cutoff value for LPC biomarker is 40 to 45 µg/ml, and a concentration of LPC in blood below the cut-off value, it can be diagnosed or determined to have lung cancer.
Also, where the cut-off value of C7 biomarker is 245 to 270 µg/ml, and the concentration of C7 biomarker in blood is above or equal to the cut-off value;
where the cutoff value for DC biomarker is 12 to 15 ng/ml, and the concentration of DC biomarker in blood is below or equal to the cutoff value;
where the cutoff value for HRG biomarker is 85 to 90 µg/ml, and the concentration of HRG biomarker in blood is below or equal to the cutoff value;
where the cutoff value for OPN biomarker is 80 to 90 ng/mL, and the concentration of OPN biomarker in blood above or equal to the cutoff value, or
where the cut-off value of LPC biomarker is 44 to 50 µg/ml, and the concentration of LPC biomarker in blood below the cut-off value, it can be diagnosed or determined to have hepatic cancer.

In the present invention,
(1) where the biomarkers are C7 and DC, the cancer can be diagnosed by the ratio of C7 concentration : DC concentration (C7/DC);
(2) where the biomarkers are C7 and LPC, the cancer can be diagnosed by the ratio of C7 concentration : LPC concentration (C7/LPC);
(3) where the biomarkers are C7 and HRG, the cancer can be diagnosed by the ratio of C7 concentration : HRG concentration (C7/HRG);
(4) where the biomarkers are C7, DC, and LPC, the cancer can be diagnosed by the ratio of C7 concentration : (sum of DC concentration and LPC concentration) (C7/(DC + LPC));
(5) where the biomarkers are C7, DC, and HRG, the cancer is diagnosed by the ratio of C7 concentration : (sum of DC concentration and HRG concentration) (C7/(DC + HRG));
(6) where the biomarkers are C7, DC, HRG, and LPC, the cancer can be diagnosed by the ratio of C7 concentration : (sum of DC concentration, HRG concentration and LPC concentration) (C7/(DC + HRG + LPC));
(7) where the biomarkers are C7, HRG, and LPC, the cancer can be diagnosed by the ratio of C7 concentration : (sum of HRG concentration and LPC concentration) (C7/(HRG + LPC));
(8) where the biomarkers are OPN and DC, the cancer can be diagnosed by the ratio of OPN concentration : DC concentration (OPN/DC);
(9) where the biomarkers are OPN and LPC, the cancer can be diagnosed by the ratio of OPN concentration : LPC concentration (OPN/LPC);
(10) where the biomarkers are OPN and HRG, the cancer can be diagnosed by the ratio of OPN concentration : HRG concentration (OPN/HRG);
(11) where the biomarkers are OPN, DC, and LPC, the cancer can be diagnosed by the ratio of OPN concentration : (sum of DC concentration and LPC concentration) (OPN/(DC + LPC)); or
(12) where the biomarkers are OPN, DC, and HRG, the cancer can be diagnosed by the ratio of OPN concentration : (sum of DC concentration and HRG concentration) (OPN/(DC + HRG)).

More specifically,
(1) where the cut-off value for the ratio of C7 concentration : DC concentration (C7/DC) is 10 to 12 and the ratio of C7 concentration : DC concentration in blood is above or equal to the cut-off value, it can be diagnosed or determined to have lung cancer;
(2) where the cut-off value for the ratio of C7 concentration : LPC concentration (C7/LPC) is 3.5 to 5 and the ratio of C7 concentration : LPC concentration in blood is above or equal to the cut-off value, it can be diagnosed or determined to have lung cancer;
(3) where the cut-off value for the ratio of C7 concentration : HRG concentration (C7/HRG) is 2 to 2.5 and the ratio of C7 concentration : HRG concentration in blood is above or equal to the cut-off value, it can be diagnosed or determined to have lung cancer;
(4) where the cut-off value for the ratio of C7 concentration : (sum of DC concentration and LPC concentration) (C7/(DC + LPC)) is 2.5 ~ 3.0 and the ratio of C7 concentration : (sum of DC concentration and LPC concentration) in blood is above or equal to the cut-off value, it can be diagnosed or determined to have lung cancer;
(5) where the cut-off value for the ratio of C7 concentration : (sum of DC concentration and HRG concentration) (C7/(DC + HRG)) is 1.5 to 2.0 and the ratio of C7 concentration : (sum of DC concentration and HRG concentration) in blood is above or equal to the cut-off value, it can be diagnosed or determined to have lung cancer;
(6) where the cut-off value for the ratio of C7 concentration : (sum of DC concentration, HRG concentration and LPC concentration) (C7/(DC + HRG + LPC)) is 1 to 1.5 and the ratio of C7 concentration : (sum of DC concentration, HRG concentration and LPC concentration) in blood, plasma or serum is above or equal to the cut-off value, it can be diagnosed or determined to have lung cancer;
(7) where the cut-off value for the ratio of C7 concentration : (sum of HRG concentration and LPC concentration) (C7/(HRG + LPC)) is 1.2 ~ 2 and the ratio of C7 concentration : (sum of HRG concentration and LPC concentration) in blood is above or equal to the cut-off value, it can be diagnosed or determined to have lung cancer;
(8) where the cut-off value for the ratio of OPN concentration : DC concentration (OPN/DC) is 3 to 5 and the ratio of OPN concentration : DC concentration in blood is above or equal to the cutoff value, it can be diagnosed or determined to have lung cancer;
(9) where the cut-off value for the ratio of OPN concentration : LPC concentration (OPN/LPC) is 1.5 to 2.0 and the ratio of OPN concentration : LPC concentration in blood is above or equal to the cut-off value, it can be diagnosed or determined to have lung cancer;
(10) where the cut-off value for the ratio of OPN concentration : HRG concentration (OPN/HRG) is 0.8 to 1.2 and the ratio of OPN concentration : HRG concentration in blood is above or equal to the cut-off value, it can be diagnosed or determined to have lung cancer;
(11) where the cut-off value for the ratio of OPN concentration : (sum of DC concentration and LPC concentration) (OPN/(DC + LPC)) is 0.9 to 1.5 and the ratio of OPN concentration : (sum of DC concentration and LPC concentration) in blood is above or equal to the cut-off value, it can be diagnosed or determined to have lung cancer; or
(12) where the cut-off value for the ratio of OPN concentration : (sum of DC concentration and HRG concentration) (OPN/(DC + HRG)) is 0.5 to 1.2 and the ratio of OPN concentration : (sum of DC concentration and HRG concentration) in blood is above or equal to the cut-off value, it can be diagnosed or determined to have lung cancer.

Also,
(1) where the cut-off value for the ratio of C7 concentration : DC concentration (C7/DC) is 8 to 12 and the ratio of C7 concentration: DC concentration ratio in blood is above or equal to the cutoff value, it can be diagnosed or determined to have hepatic cancer;
(2) where the cut-off value for the ratio of C7 concentration : LPC concentration ratio (C7/LPC) is 3.5 to 5 and the ratio of C7 concentration : LPC concentration in blood is above or equal to the cut-off value, it can be diagnosed or determined to have hepatic cancer;
(3) where the cut-off value for the ratio of C7 concentration : HRG concentration (C7/HRG) is 2 to 2.5 and the ratio of C7 concentration : HRG concentration in blood is above or equal to the cut-off value, it can be diagnosed or determined to have hepatic cancer;
(4) where the cut-off value for the ratio of C7 concentration : (sum of DC concentration and LPC concentration) (C7/(DC + LPC)) is 2 to 4 and the ratio of C7 concentration : (sum of DC concentration and LPC concentration) in blood is above or equal to the cut-off value, it can be diagnosed or determined to have hepatic cancer;
(5) where the cut-off value for the ratio of C7 concentration : (sum of DC concentration and HRG concentration) (C7/(DC + HRG)) is 1.5 ~ 2 and the ratio of C7 concentration : (sum of DC concentration and HRG concentration) in blood is above or equal to the cut-off value, it can be diagnosed or determined to have hepatic cancer;
(6) where the cut-off value for the ratio of C7 concentration : (sum of DC concentration, HRG concentration, and LPC concentration) (C7/(DC + HRG + LPC)) is 1.2 to 2 and the ratio of C7 concentration : (sum of DC concentration, HRG concentration, and LPC concentration) in blood is above or equal to the cut-off value, it can be diagnosed or determined to have hepatic cancer;
(7) where the cut-off value for the ratio of C7 concentration : (sum of HRG concentration and LPC concentration) (C7/(HRG + LPC)) is 1 to 3 and the ratio of C7 concentration : (sum of HRG concentration and LPC concentration) in blood is above or equal to the cut-off value, it can be diagnosed or determined to have hepatic cancer;
(8) where the cut-off value for the ratio of OPN concentration : DC concentration (OPN/DC) is 3 to 5 and the ratio of OPN concentration: DC concentration in blood is above or equal to the cutoff value, it can be diagnosed or determined to have hepatic cancer;
(9) where the cut-off value for the ratio of OPN concentration : LPC concentration (OPN/LPC) is 1.5 to 2.5 and the ratio of OPN concentration : LPC concentration in blood is above or equal to the cut-off value, it can be diagnosed or determined to have hepatic cancer;
(10) where the cut-off value for the ratio of OPN to HRG (OPN/HRG) is 3 to 5 and the ratio of OPN concentration : HRG concentration in blood is above or equal to the cut-off value, it can be diagnosed or determined to have hepatic cancer;
(11) where the cut-off value for the ratio of OPN concentration : (sum of DC concentration and LPC concentration) (OPN/(DC + LPC)) is 0.8 ~ 2 and the ratio of OPN concentration : (sum of DC concentration and LPC concentration) in blood is above or equal to the cut-off value, it can be diagnosed or determined to have hepatic cancer; or
(12) where the cut-off value for the ratio of OPN concentration : (sum of DC concentration and HRG concentration) (OPN/(DC + HRG)) is 0.5 to 1.2 and the ratio of OPN concentration : (sum of DC concentration and HRG concentration) in blood is above or equal to the cut-off value, it can be diagnosed or determined to have hepatic cancer.

### Composition for diagnosing cancer

In another aspect, the present invention relates to a composition for diagnosing cancer, comprising an agent for measuring levels in blood of a biomarker composition for diagnosing cancer of the present invention.

The composition for diagnosing cancer according to the present invention can refer to "Biomarker Composition for diagnosing cancer" above.

In the present invention, an agent for measuring the level of C7, HRG or OPN biomarker may be an agent for measuring protein levels, and an agent for measuring the level of DC biomarker or LPC biomarker may be an agent for mass spectrometry.

The agent for measuring the protein levels may be an antibody, interacting protein, ligand, nanoparticle, or aptamer that specifically binds to a protein or peptide fragment of C7, HRG, or OPN marker.

As used in the present invention, the term "antibody" refers to an agent that specifically binds to an antigen and causes an antigen-antibody reaction. For the purposes of the present invention, an antibody is an antibody that binds specifically to a biomarker of the present invention. Antibodies of the present invention include polyclonal antibodies, monoclonal antibodies, and recombinant antibodies.

As used in the present invention, the term "aptamer" refers to a biopolymeric material in the form of single- or double-stranded DNA or RNA that inhibits protein interactions through three-dimensional binding to a specific target protein, characterized by binding to a variety of target molecules. Typically, aptamers can be small nucleic acids of 15 to 50 bases in length that fold into a defined secondary and tertiary structure, such as a stem-loop structure. Preferably, the aptamer binds the targeted high or low expression protein with a KD less than 10⁻⁶, 10⁻⁸, 10⁻¹⁰, or 10⁻¹².

The agent for mass spectrometry means an agent or a preparation capable of analyzing the mass of a biomarker in blood, plasma or serum, more specifically, an agent or a preparation capable of performing liquid chromatography-mass spectrometry (LC-MS).

### Kit for diagnosing cancer

In another aspect, the present invention relates to a kit for diagnosing cancer comprising an agent for measuring levels in blood of a biomarker composition for diagnosing cancer of the present invention.

The kit for diagnosing cancer according to the present invention can refer to "Biomarker Composition for diagnosing cancer" above.

The kit may be prepared by conventional methods known in the art. The kit may include, for example, antibodies in lyophilized form and buffers, stabilizers, inactive proteins, and the like.

The kit may further comprise a detectable label. The term "detectable label" means an atom or molecule that enables the specific detection of a molecule comprising the label among molecules of the same kind that are unlabeled. The detectable label may be attached to an antibody, interacting protein, ligand, nanoparticle, or aptamer that specifically binds to the protein or fragment thereof, the detectable label may comprise a radionuclide, a fluorophore, or an enzyme.

The kit can be utilized according to various immunoassays or immunostaining methods known in the art. Such immunoassays or immunostaining methods may include radioimmunoassays, radioimmunoprecipitation, immunoprecipitation, ELISA, capture-ELISA, inhibition or competition assays, sandwich assays, flow cytometry, immunofluorescence staining, and immunoaffinity purification. Preferably, the kit may be an enzyme linked immunosorbent assay (ELISA) kit, protein chip kit, rapid kit, or multiple reaction monitoring (MRM).

### Method of providing information for diagnosing cancer

In another aspect, the present invention provides a method of providing information for diagnosing cancer, comprising (a) measuring levels of one or more biomarkers selected from the group consisting of Complement Component 7 (C7), Dodecanoyl-L-carnitine (DC), Lysophosphatidylcholine (LPC), Histidine-Rich Glycoprotein (HRG), and Osteopontin (OPN) in the patient's blood; and (b) comparing levels of the biomarker to those from a normal control sample.

In the present invention, the cancer may be lung cancer, pancreatic cancer, biliary tract cancer, colorectal cancer, breast cancer, gastric cancer, brain tumor, kidney cancer, hepatic cancer or cervical cancer, preferably hepatic cancer or lung cancer.

In the present invention, the lysophosphatidylcholine (LPC) may be lysophosphatidylcholine 16:0 (LPC16) or lysophosphatidylcholine 18:0 (LPC18).

In the present invention, the method of providing information for diagnosing cancer may further comprise the step of providing information that cancer is present,
where the concentration of C7 biomarker in blood is higher than that of a normal control;
where the concentration of OPN biomarker in blood is higher than that of a normal control;
where the concentration of DC biomarker in blood is lower than that of a normal control;
where the concentration of LPC biomarker in blood is lower than that of a normal control; or
where the concentration of Histidine-Rich Glycoprotein (HRG) biomarker in blood is lower than that of a normal control group.

Specifically, the method of providing information for diagnosing cancer may provide information that lung cancer is present,
where the cut-off value of C7 biomarker is 230 to 240 µg/ml, and the concentration of C7 in blood is above or equal to the cut-off value;
where the cutoff value for DC biomarker is 14 to 17 ng/mL, and the concentration of DC in blood is below or equal to the cutoff value;
where the cutoff value for HRG biomarker is 90 to 95 µg/ml, and a concentration of HRG in blood below or equal to the cutoff value; or
where the cutoff value for LPC biomarker is 40 to 45 µg/ml, and a concentration of LPC in blood below the cut-off value.
Also, the method of providing information for diagnosing cancer may provide information that hepatic cancer is present,
where the cut-off value of C7 biomarker is 245 to 270 µg/ml, and the concentration of C7 biomarker in blood is above or equal to the cut-off value;
where the cutoff value for DC biomarker is 12 to 15 ng/ml, and the concentration of DC biomarker in blood is below or equal to the cutoff value;
where the cutoff value for HRG biomarker is 85 to 90 µg/ml, and the concentration of HRG biomarker in blood is below or equal to the cutoff value;
where the cutoff value for OPN biomarker is 80 to 90 ng/mL, and the concentration of OPN biomarker in blood above or equal to the cutoff value, or
where the cut-off value of LPC biomarker is 44 to 50 µg/ml, and the concentration of LPC biomarker in blood below the cut-off value.

In the present invention,
(1) where the biomarkers are C7 and DC, the cancer is diagnosed by the ratio of C7 concentration : DC concentration (C7/DC);
(2) where the biomarkers are C7 and LPC, the information for diagnosing cancer is measured or provided by the ratio of C7 concentration : LPC concentration (C7/LPC);
(3) where the biomarkers are C7 and HRG, the information for diagnosing cancer is measured or provided by the ratio of C7 concentration : HRG concentration (C7/HRG);
(4) where the biomarkers are C7, DC, and LPC, the information for diagnosing cancer is measured or provided by the ratio of C7 concentration : (sum of DC concentration and LPC concentration) (C7/(DC + LPC));
(5) where the biomarkers are C7, DC, and HRG, the information for diagnosing cancer is measured or provided by the ratio of C7 concentration : (sum of DC concentration and HRG concentration) (C7/(DC + HRG));
(6) where the biomarkers are C7, DC, HRG, and LPC, the information for diagnosing cancer is measured or provided by the ratio of C7 concentration : (sum of DC concentration, HRG concentration and LPC concentration) (C7/(DC + HRG + LPC));
(7) where the biomarkers are C7, HRG, and LPC, the information for diagnosing cancer is measured or provided by the ratio of C7 concentration : (sum of HRG concentration and LPC concentration) (C7/(HRG + LPC));
(8) where the biomarkers are OPN and DC, the information for diagnosing cancer is measured or provided by the ratio of OPN concentration : DC concentration (OPN/DC);
(9) where the biomarkers are OPN and LPC, the information for diagnosing cancer is measured or provided by the ratio of OPN concentration : LPC concentration (OPN/LPC);
(10) where the biomarkers are OPN and HRG, the information for diagnosing cancer is measured or provided by the ratio of OPN concentration : HRG concentration (OPN/HRG);
(11) where the biomarkers are OPN, DC, and LPC, the information for diagnosing cancer is measured or provided by the ratio of OPN concentration : (sum of DC concentration and LPC concentration) (OPN/(DC + LPC)); or
(12) where the biomarkers are OPN, DC, and HRG, the information for diagnosing cancer is measured or provided by the ratio of OPN concentration : (sum of DC concentration and HRG concentration) (OPN/(DC + HRG)).

Specifically,
(1) where the cut-off value for the ratio of C7 concentration : DC concentration (C7/DC) is 10 to 12 and the ratio of C7 concentration : DC concentration in blood is above or equal to the cut-off value, the cancer is lung cancer;
(2) where the cut-off value for the ratio of C7 concentration : LPC concentration (C7/LPC) is 3.5 to 5 and the ratio of C7 concentration : LPC concentration in blood is above or equal to the cut-off value, the cancer is lung cancer;
(3) where the cut-off value for the ratio of C7 concentration : HRG concentration (C7/HRG) is 2 to 2.5 and the ratio of C7 concentration : HRG concentration in blood is above or equal to the cut-off value, the cancer is lung cancer;
(4) where the cut-off value for the ratio of C7 concentration : (sum of DC concentration and LPC concentration) (C7/(DC + LPC)) is 2.5 ~ 3.0 and the ratio of C7 concentration : (sum of DC concentration and LPC concentration) in blood is above or equal to the cut-off value, the cancer is lung cancer;
(5) where the cut-off value for the ratio of C7 concentration : (sum of DC concentration and HRG concentration) (C7/(DC + HRG)) is 1.5 to 2.0 and the ratio of C7 concentration : (sum of DC concentration and HRG concentration) in blood is above or equal to the cut-off value, the cancer is lung cancer;
(6) where the cut-off value for the ratio of C7 concentration : (sum of DC concentration, HRG concentration and LPC concentration) (C7/(DC + HRG + LPC)) is 1 to 1.5 and the ratio of C7 concentration : (sum of DC concentration, HRG concentration and LPC concentration) in blood, plasma or serum is above or equal to the cut-off value, the cancer is lung cancer;
(7) where the cut-off value for the ratio of C7 concentration : (sum of HRG concentration and LPC concentration) (C7/(HRG + LPC)) is 1.2 ~ 2 and the ratio of C7 concentration : (sum of HRG concentration and LPC concentration) in blood is above or equal to the cut-off value, the cancer is lung cancer;
(8) where the cut-off value for the ratio of OPN concentration : DC concentration (OPN/DC) is 3 to 5 and the ratio of OPN concentration : DC concentration in blood is above or equal to the cutoff value, the cancer is lung cancer;
(9) where the cut-off value for the ratio of OPN concentration : LPC concentration (OPN/LPC) is 1.5 to 2.0 and the ratio of OPN concentration : LPC concentration in blood is above or equal to the cut-off value, the cancer is lung cancer;
(10) where the cut-off value for the ratio of OPN concentration : HRG concentration (OPN/HRG) is 0.8 to 1.2 and the ratio of OPN concentration : HRG concentration in blood is above or equal to the cut-off value, the cancer is lung cancer;
(11) where the cut-off value for the ratio of OPN concentration : (sum of DC concentration and LPC concentration) (OPN/(DC + LPC)) is 0.9 to 1.5 and the ratio of OPN concentration : (sum of DC concentration and LPC concentration) in blood is above or equal to the cut-off value, the cancer is lung cancer; or
(12) where the cut-off value for the ratio of OPN concentration : (sum of DC concentration and HRG concentration) (OPN/(DC + HRG)) is 0.5 to 1.2 and the ratio of OPN concentration : (sum of DC concentration and HRG concentration) in blood is above or equal to the cut-off value, the cancer is lung cancer.

Also,
(1) where the cut-off value for the ratio of C7 concentration : DC concentration (C7/DC) is 8 to 12 and the ratio of C7 concentration: DC concentration ratio in blood is above or equal to the cutoff value, the cancer is hepatic cancer;
(2) where the cut-off value for the ratio of C7 concentration : LPC concentration ratio (C7/LPC) is 3.5 to 5 and the ratio of C7 concentration : LPC concentration in blood is above or equal to the cut-off value, the cancer is hepatic cancer;
(3) where the cut-off value for the ratio of C7 concentration : HRG concentration (C7/HRG) is 2 to 2.5 and the ratio of C7 concentration : HRG concentration in blood is above or equal to the cut-off value, the cancer is hepatic cancer;
(4) where the cut-off value for the ratio of C7 concentration : (sum of DC concentration and LPC concentration) (C7/(DC + LPC)) is 2 to 4 and the ratio of C7 concentration : (sum of DC concentration and LPC concentration) in blood is above or equal to the cut-off value, the cancer is hepatic cancer;
(5) where the cut-off value for the ratio of C7 concentration : (sum of DC concentration and HRG concentration) (C7/(DC + HRG)) is 1.5 ~ 2 and the ratio of C7 concentration : (sum of DC concentration and HRG concentration) in blood is above or equal to the cut-off value, the cancer is hepatic cancer;
(6) where the cut-off value for the ratio of C7 concentration : (sum of DC concentration, HRG concentration, and LPC concentration) (C7/(DC + HRG + LPC)) is 1.2 to 2 and the ratio of C7 concentration : (sum of DC concentration, HRG concentration, and LPC concentration) in blood is above or equal to the cut-off value, the cancer is hepatic cancer;
(7) where the cut-off value for the ratio of C7 concentration : (sum of HRG concentration and LPC concentration) (C7/(HRG + LPC)) is 1 to 3 and the ratio of C7 concentration : (sum of HRG concentration and LPC concentration) in blood is above or equal to the cut-off value, the cancer is hepatic cancer;
(8) where the cut-off value for the ratio of OPN concentration : DC concentration (OPN/DC) is 3 to 5 and the ratio of OPN concentration: DC concentration in blood is above or equal to the cutoff value, the cancer is hepatic cancer;
(9) where the cut-off value for the ratio of OPN concentration : LPC concentration (OPN/LPC) is 1.5 to 2.5 and the ratio of OPN concentration : LPC concentration in blood is above or equal to the cut-off value, the cancer is hepatic cancer;
(10) where the cut-off value for the ratio of OPN to HRG (OPN/HRG) is 3 to 5 and the ratio of OPN concentration : HRG concentration in blood is above or equal to the cut-off value, the cancer is hepatic cancer;
(11) where the cut-off value for the ratio of OPN concentration : (sum of DC concentration and LPC concentration) (OPN/(DC + LPC)) is 0.8 ~ 2 and the ratio of OPN concentration : (sum of DC concentration and LPC concentration) in blood is above or equal to the cut-off value, the cancer is hepatic cancer; or
(12) where the cut-off value for the ratio of OPN concentration : (sum of DC concentration and HRG concentration) (OPN/(DC + HRG)) is 0.5 to 1.2 and the ratio of OPN concentration : (sum of DC concentration and HRG concentration) in blood is above or equal to the cut-off value, the cancer is hepatic cancer.

In the present invention, the measuring levels of one or more biomarkers selected from the group consisting of C7, HRG and OPN in step (a) above can be performed using protein chip analysis, immunometry, ligand binding assay, matrix desorption/ionization time of flight mass spectrometry (MALDI-TOF) analysis, surface enhanced laser desorption/ionization time of flight mass spectrometry (SELDI-TOF) analysis, radioimmunoassay, radioimmunodiffusion, or ouchterlony immunodiffusion, rocket immunoelectrophoresis, tissue immunostaining, complement fixation assay, two-dimensional electrophoresis analysis, liquid chromatography-mass spectrometry (LC-MS), liquid chromatography-mass spectrometry/mass spectrometry (LC-MS/MS), western blot, and/or enzyme linked immunosorbent assay (ELISA).

In the present invention, the measuring levels of one or more biomarkers selected from the group consisting of DC and LPC in step (a) above can be obtained by mass spectrometry of the sample in blood, plasma or serum by mass peak area, more specifically by liquid chromatography-mass spectrometry (LC-MS). The mass spectrometer may be any one of Triple TOF, Triple Quadrupole and MALDI TOF capable of quantitative measurements.

### Biomarker for cancer prognosis

In another aspect, the present invention provides a composition for predicting cancer prognosis, wherein the composition comprises at least one biomarker selected from the group consisting of Complement Component 7 (C7), Dodecanoyl-L-carnitine (DC), Lysophosphatidylcholine (LPC), Histidine-Rich Glycoprotein (HRG), and Osteopontin (OPN), and wherein the biomarker is derived from blood.

In the present invention, where the concentration of C7 biomarker in blood is higher than that of a normal control;
where the concentration of OPN biomarker in blood is higher than that of a normal control;
where the concentration of DC biomarker in blood is lower than that of a normal control;
where the concentration of LPC biomarker in blood is lower than that of a normal control; or
where the concentration of Histidine-Rich Glycoprotein (HRG) biomarker in blood is lower than that of a normal control group, it can be diagnosed or determined to poor prognosis of cancer. The details can refer to "Biomarker Composition for diagnosing cancer" above.

### Composition and kit for predicting cancer prognosis

In another aspect, the present invention relates to a composition for predicting cancer prognosis, comprising an agent for measuring levels in blood of the biomarker composition for predicting cancer prognosis.

In another aspect, the present invention relates to a kit for diagnosing cancer prognosis, comprising a composition for predicting cancer prognosis.

Specific details of the composition or kit for predicting cancer prognosis according to the present invention refer to "Composition for diagnosing cancer" or "Kit for diagnosing cancer" above.

### Method of providing information for predicting cancer prognosis

In another aspect, the present invention provides a method of providing information for predicting cancer prognosis, comprising: comprising (a) measuring levels of one or more biomarkers selected from the group consisting of Complement Component 7 (C7), Dodecanoyl-L-carnitine (DC), Lysophosphatidylcholine (LPC), Histidine-Rich Glycoprotein (HRG), and Osteopontin (OPN) in the patient's blood; and (b) comparing levels of the biomarker to those from a normal control sample.

In the present invention, where the concentration of C7 biomarker in blood is higher than that of a normal control;
where the concentration of OPN biomarker in blood is higher than that of a normal control;
where the concentration of DC biomarker in blood is lower than that of a normal control;
where the concentration of LPC biomarker in blood is lower than that of a normal control; or
where the concentration of Histidine-Rich Glycoprotein (HRG) biomarker in blood is lower than that of a normal control group, it can be diagnosed or determined to poor prognosis of cancer. The details can refer to "Method of providing information for diagnosing cancer" above.

The present invention will now be described in more detail with reference to the following examples.

These embodiments are intended solely to illustrate the invention, and it will be apparent to one of ordinary skill in the art that the scope of the invention is not to be construed as limited by these embodiments.

### Example 1: Diagnosing a patient with lung cancer

### 1-1: Sample preparation

To whether the biomarker of the present invention can diagnose lung cancer, a total of 189 patients were screened, specifically, 89 normal people and 100 patients with lung cancer (LC) were screened and blood was collected.

### 1-2: Measurement of biomarker concentrations in blood

### <Measurement of Complement Component C7 (C7) concentration>

Plasma was extracted from each of the above blood samples, and C7 enzyme-linked immunosorbent assay (ELISA) was performed to measure the amount of C7 protein. C7 ELISA kit was the YN1203 ELISA Kit (INNOBATION BIO, Korea).

The isolated plasma was diluted 200,000-fold using the diluent in the ELISA kit. Next, the standards for C7 standard curve were prepared according to the manufacturer's manual. The prepared samples and standards were placed in a plate coated with C7 antibody, sealed, and incubated at room temperature for 2 hours.

After 2 hours of incubation, the contents of each plate well were removed and washed three times with washing solution. After completion of washing, the plates were loaded with detection antibodies and incubated at room temperature for 1 hour.

After 1 hour of incubation, the contents of each plate well were removed and washed three times with washing solution. After completion of washing, substrate was added to the plate and incubated at room temperature for 20 minutes.

After 20 minutes of incubation, the stop solution was added to each plate well and mixed by gentle tapping, and the absorbance was measured using a microplate reader (Medical ImmunoAbsorbometer, K01050.01) set at 450 nm. The measured absorbance was plotted against a standard curve prepared with standards to determine the appropriate concentration of C7 protein.

### <Measurement of Dodecanoyl-L-carnitine (DC) concentration>

Plasma was separated from the above blood samples, and standards were prepared at the concentrations required for DC (Sigma, USA) standard curve. 20 µl of plasma and standards were added to 400 µl of lipid extraction buffer (Abnova, Taiwan), vortexed, and centrifuged (10,000g, 5 min, 4°C). After centrifugation, the supernatant was transferred to a new tube and dried using a concentrator for 12 to 16 hours. To the dried metabolite extract, 50 µl of 100% methanol with 0.1% formic acid was added, dissolved well using a vortex, and analyzed using liquid chromatography-mass spectrometry/mass spectrometry (LC-MS/MS).

The LC used was a Shimadzu LC 40 system and the MS was an AB Sciex Triple Quad 5500+ system. The MS was equipped with a Turbo Spray ion source. The analytes were separated within a BEH C18 column (1.7 um, 2.1×50 mm; Waters) on a Shimadzu LC 40 system. The solvent was a two-step linear gradient (solvent A, 0.1% FA in water; solvent B, 0.1% FA in 100% Acetonitrile; 5 to 55% solvent B 2.5 min, 55% solvent B 5.5 min, 55 to 95% solvent B 7.5 min, 95% solvent B 11 min, 95 to 5% solvent B 11.1 min, and 5% solvent B 14.5 min).

Mass spectrometry (MS/MS) was performed using multiple reaction monitoring (MRM) mode. The area of the mass peak with the same mass value in the mass spectrum of the same time period as the time period where the metabolite corresponding to DC passed through the liquid chromatography was calculated. A standard curve was constructed using the mass peaks of DC standards, and the concentration of DC was determined by fitting the mass peaks of each sample to the standard curve.

### <Measurement of LPC16 concentration>

Plasma was separated from the above blood samples, and standards were prepared at the concentrations required for LPC (Avanti, USA) standard curve. 20 µl of plasma and standards were vortexed with 400 µl of lipid extraction buffer (Abnova, Taiwan) and centrifuged (10,000g, 5min, 4°C) after vortexing.

After centrifugation, the supernatant was transferred to a new tube and allowed to dry for 12-16 hours using a concentrator. To the dried metabolite extract, 50 µl of 100% methanol with 0.1% formic acid was added, vortexed to dissolve well, and analyzed using liquid chromatography-mass spectrometry/mass spectrometry (LC-MS/MS).

The LC used was a Shimadzu LC 40 system and the MS was an AB Sciex Triple Quad 5500+ system. The MS was equipped with a Turbo Spray ion source. The analytes were separated within a BEH C18 column (1.7 um, 2.1×50 mm; Waters) on a Shimadzu LC 40 system. The solvent was a two-step linear gradient (solvent A, 0.1% FA in water; solvent B, 0.1% FA in 100% Acetonitrile; 5 to 55% solvent B 2.5 min, 55% solvent B 5.5 min, 55 to 95% solvent B 7.5 min, 95% solvent B 11 min, 95 to 5% solvent B 11.1 min, and5% solvent B 14.5 min).

Mass spectrometry (MS/MS) was performed using multiple reaction monitoring (MRM) mode. The area of the mass peak with the same mass value in the mass spectrum of the same time period as the time where the metabolite corresponding to LPC passed through liquid chromatography was calculated. A standard curve was constructed using the mass peaks of LPC standards, and the concentration of LPC was determined by fitting the mass peaks of each sample to the standard curve.

### <Measurement of Histidine-Rich Glycoprotein (HRG) Concentration>

Plasma was extracted from each of the above blood samples and HRG enzyme-linked immunosorbent assay (ELISA) was performed to determine the amount of HRG protein. HRG ELISA kit was from My BioSource (USA).

The isolated plasma was diluted 100-fold using the diluent in the ELISA kit. Next, the standards for HRG standard curve were prepared according to the manufacturer's manual. The prepared samples and standards were added to the plate coated with HRG antibody, sealed, and incubated at room temperature for 1 hour. After 1 hour of incubation, the contents of each plate well were removed and washed three times with washing solution, and the plate was incubated with detection antibody for 1 hour at room temperature. After 1 hour of incubation, the contents of each plate well were removed and washed three times with washing solution, and the plate was incubated at room temperature for 30 minutes after adding substrate to the plate after completion of washing.

After 30 minutes of incubation, the stop solution was added to each plate well and gently tapped to mix, and the absorbance was measured using a microplate reader (Medical ImmunoAbsorbometer, K01050.01) set at 450 nm. The measured absorbance was converted to the appropriate amount of HRG protein by fitting a standard curve prepared with standards.

### <Measurement of Osteopontin Concentration>

After extracting plasma from each of the above blood samples, osteopontin enzyme-linked immunosorbent assay (ELISA) was performed to measure the amount of osteopontin protein. The osteopontin ELISA kit was the Osteopontin Human ELISA Kit (Invitrogen, USA).

The isolated plasma was diluted 20-fold with the diluent in the ELISA kit. Before use, the ELISA kit was washed three times with washing solution, and the standards for osteopontin standardization curve were prepared according to the manufacturer's manual. The prepared samples and standards were added to the plate coated with osteopontin antibody, sealed, and incubated at 60 rpm at room temperature for 2 hours.

After 2 hours of incubation, the contents of each plate well were removed and washed three times with washing solution, and after completion of washing, the plate was loaded with Biotin-Conjugate and incubated at room temperature for 1 hour. After 1 hour of incubation, the contents of each plate well were removed and washed three times with washing solution, and after completion of washing, the plate was incubated with Streptavidin-HRP for 1 hour at room temperature. After 1 hour of incubation, the contents of each plate well were removed and washed three times with washing solution. After washing, the plate was loaded with 100 µl of substrate and incubated at room temperature for 30 minutes.

After 30 minutes of incubation, 100 µl of stop solution was added to each plate well, mixed by gentle tapping, and the absorbance was measured by setting the microplate reader (Medical Immunoabsorbance Meter, K01050.01) to 450 nm. The measured absorbance was converted to the appropriate amount of osteopontin protein by fitting a standard curve prepared with standards.

As a result, as shown in FIG. 1(A) to FIG. 1(D), we found that lung cancer patients have different concentrations of markers in their blood than normal people, specifically, lung cancer patients have higher concentrations of C7 and lower concentrations of DC, LPC, or HRG compared to normal people.

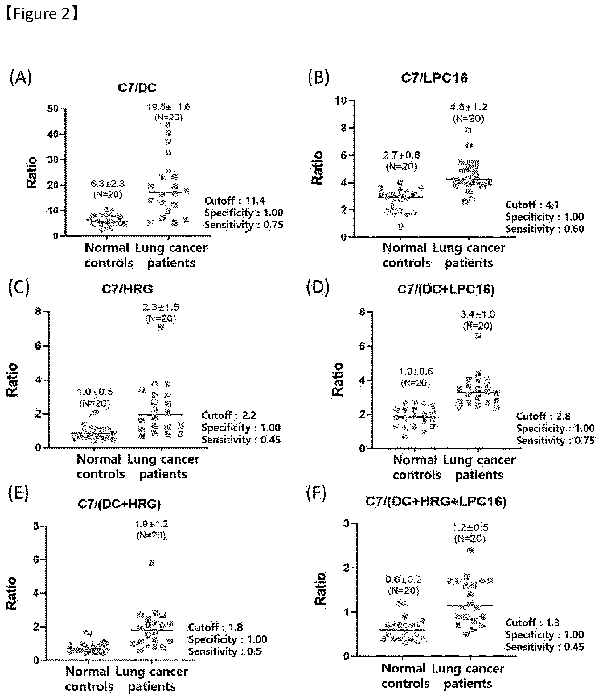

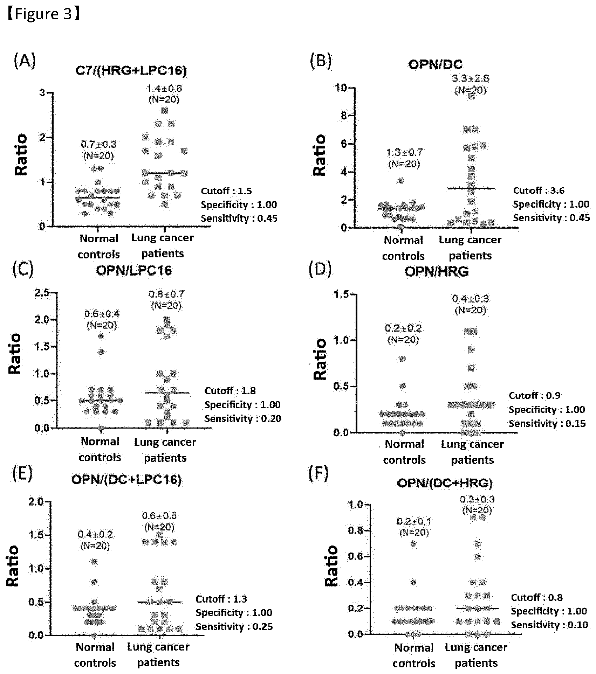

In addition, when using two or more types of markers, such as "combination of C7 and DC markers", "combination of C7 and LPC markers", "combination of C7 and HRG markers", "combination of C7, DC and LPC markers", "combination of C7, DC and HRG markers", "combination of C7, DC, HRG and LPC markers", "combination of C7, HRG and LPC markers", "combination of OPN and DC markers", "combination of OPN and LPC markers", "combination of OPN and HRG markers", "combination of OPN, DC and LPC markers", or "combination of OPN, DC and HRG markers", a clear difference in concentration ratios was observed between normal controls and lung cancer patients, as shown in FIGS. 2(A) to 2(F) and FIGS. 3(A) to 3(F). In other words, we found that the diagnostic ability of lung cancer was significantly increased by combining two or more markers rather than markers alone.

### Example 2: Diagnosing a patient with hepatic cancer

### 2-1: Sample Preparation

To determine whether the biomarker of the present invention can diagnose hepatic cancer, a total of 117 patients were screened, specifically, 100 normal and 17 patients with hepatic cancer (HC) were screened and blood was collected.

### 2-2: Measuring biomarker concentrations in blood

### <Measurement of Complement Component C7 (C7) concentration>

An enzyme-linked immunosorbent assay (ELISA) was performed in the same manner as in Example 1 above to determine the amount of C7 protein.

### <Measurement of Dodecanoyl-L-carnitine (DC) concentration>

Liquid chromatography was performed using the same method as in Example 1 above, and the area of the mass peak with the same mass value in the mass spectrum of the same time period as the time period where the metabolite corresponding to DC passed through the liquid chromatography was calculated. A standard curve was constructed using the mass peaks of DC standards, and the mass peaks of each sample were plotted against the standard curve to determine the concentration of DC.

### <Measurement of LPC16 concentration>

Liquid chromatography was performed using the same method as in Example 1 above, and the area of the mass peak with the same mass value in the mass spectrum at the same time period as the time where the metabolite corresponding to LPC passed through the liquid chromatography was calculated. A standard curve was constructed using the mass peaks of LPC standards, and the concentration of LPC was determined by substituting the mass peaks of each sample into the standard curve.

### <Measurement of Histidine-Rich Glycoprotein (HRG) Concentration>

An enzyme-linked immunosorbent assay (ELISA) was performed using the same method as in Example 1 above to determine the amount of HRG protein.

### <Measurement of Osteopontin Concentration>

An enzyme-linked immunosorbent assay (ELISA) was performed in the same manner as in Example 1 above to determine the amount of osteopontin protein.

As a result, as shown in FIGS. 4(A) through 4(E), we found that hepatic cancer patients have different concentrations of markers in their blood compared to normal individuals, specifically, higher concentrations of C7 and OPN and lower concentrations of DC, LPC, or HRG compared to normal individuals.

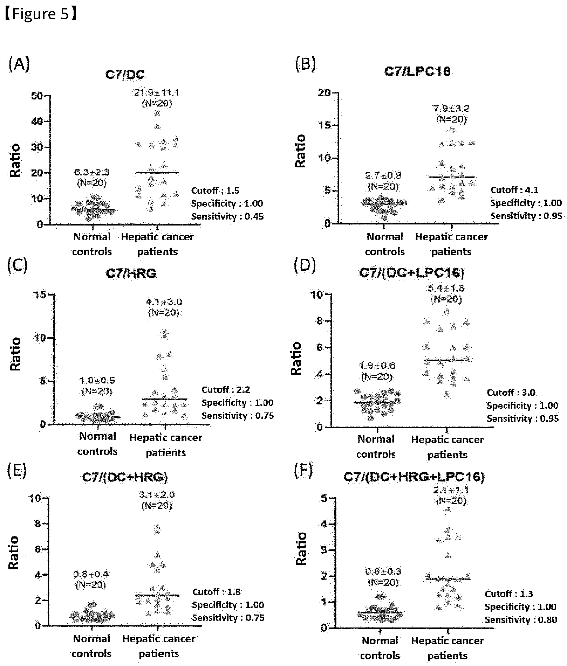

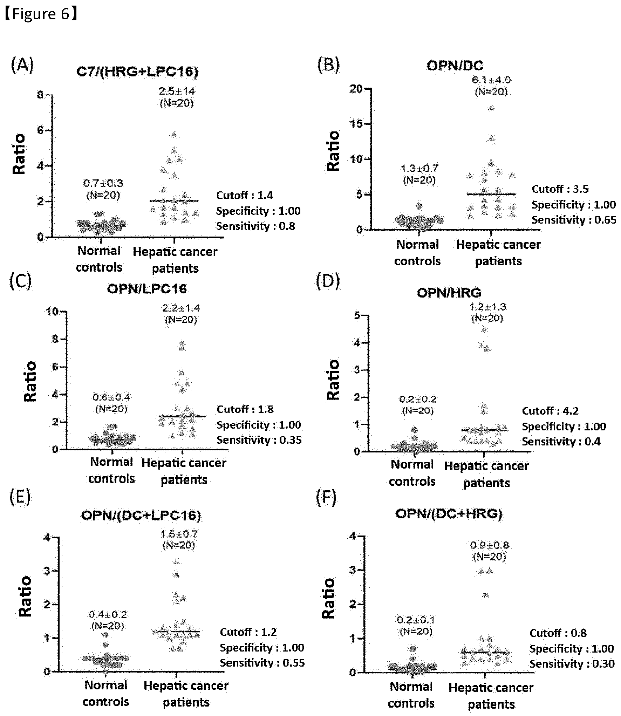

In addition, when using two or more types of markers, such as "combination of C7 and DC markers", "combination of C7 and LPC markers", "combination of C7 and HRG markers", "combination of C7, DC, and LPC markers", "combination of C7, DC, and HRG markers", "combination of C7, DC, HRG, and LPC markers", "combination of C7, HRG, and LPC markers", "combination of OPN and DC markers", "combination of OPN and LPC markers", "combination of OPN and HRG markers", "combination of OPN, DC and LPC markers", or "combination of OPN, DC and HRG markers", a clear difference in concentration ratio was observed between normal controls and HC patients, as shown in FIGS. 5(A) to 5(F) and FIGS. 6(A) to 6(F). In other words, we found that the diagnostic ability of hepatic cancer was significantly increased by combining two or more markers rather than markers alone.

The markers of the present invention may be useful in the diagnosis and prognosis of cancer, as we also have found that the combination of markers comprising Complement Component 7 (C7), Dodecanoyl-L-carnitine (DC), Lysophosphatidylcholine (LPC), Histidine-Rich Glycoprotein (HRG), and Osteopontin (OPN) in blood of lung and hepatic cancer patients improves the diagnosis of various cancers, including lung or hepatic cancer.

## Claims

1. A biomarker composition for diagnosing cancer,
wherein the composition comprises at least one biomarker selected from the group consisting of Complement Component 7 (C7), Dodecanoyl-L-carnitine (DC), Lysophosphatidylcholine (LPC), Histidine-Rich Glycoprotein (HRG), and Osteopontin (OPN), and wherein the biomarker is derived from blood.

2. The biomarker composition for diagnosing cancer of claim 1, wherein blood is whole blood, plasma, or serum.

3. The biomarker composition for diagnosing cancer of claim 1, wherein the cancer is lung cancer, pancreatic cancer, biliary tract cancer, colorectal cancer, breast cancer, gastric cancer, brain tumor, kidney cancer, hepatic cancer, or cervical cancer.

4. The biomarker composition for diagnosing cancer of claim 1, wherein the lysophosphatidylcholine (LPC) is lysophosphatidylcholine 16:0 (LPC16) or lysophosphatidylcholine 18:0 (LPC18).

5. The biomarker composition for diagnosing cancer of claim 1,
where the concentration of C7 biomarker in blood is higher than that of a normal control;
where the concentration of OPN biomarker in blood is higher than that of a normal control;
where the concentration of DC biomarker in blood is lower than that of a normal control;
where the concentration of LPC biomarker in blood is lower than that of a normal control; or
where the concentration of Histidine-Rich Glycoprotein (HRG) biomarker in blood is lower than that of a normal control group,
it is diagnosed or determined to have cancer.

6. The biomarker composition for diagnosing cancer of claim 5,
where the cut-off value of C7 biomarker is 230 to 240 µg/ml, and the concentration of C7 in blood is above or equal to the cut-off value;
where the cutoff value for DC biomarker is 14 to 17 ng/mL, and the concentration of DC in blood is below or equal to the cutoff value;
where the cutoff value for HRG biomarker is 90 to 95 µg/ml, and a concentration of HRG in blood below or equal to the cutoff value; or
where the cutoff value for LPC biomarker is 40 to 45 µg/ml, and a concentration of LPC in blood below the cut-off value,
it is diagnosed or determined to have lung cancer.

7. The biomarker composition for diagnosing cancer of claim 5,
where the cut-off value of C7 biomarker is 245 to 270 µg/ml, and the concentration of C7 biomarker in blood is above or equal to the cut-off value;
where the cutoff value for DC biomarker is 12 to 15 ng/ml, and the concentration of DC biomarker in blood is below or equal to the cutoff value;
where the cutoff value for HRG biomarker is 85 to 90 µg/ml, and the concentration of HRG biomarker in blood is below or equal to the cutoff value;
where the cutoff value for OPN biomarker is 80 to 90 ng/mL, and the concentration of OPN biomarker in blood above or equal to the cutoff value, or
where the cut-off value of LPC biomarker is 44 to 50 µg/ml, and the concentration of LPC biomarker in blood below the cut-off value,
it is diagnosed or determined to have hepatic cancer.

8. The biomarker composition for diagnosing cancer of claim 1,
(1) where the biomarkers are C7 and DC, the cancer is diagnosed by the ratio of C7 concentration : DC concentration (C7/DC);
(2) where the biomarkers are C7 and LPC, the cancer is diagnosed by the ratio of C7 concentration : LPC concentration (C7/LPC);
(3) where the biomarkers are C7 and HRG, the cancer is diagnosed by the ratio of C7 concentration : HRG concentration (C7/HRG);
(4) where the biomarkers are C7, DC, and LPC, the cancer is diagnosed by the ratio of C7 concentration : (sum of DC concentration and LPC concentration) (C7/(DC + LPC));
(5) where the biomarkers are C7, DC, and HRG, the cancer is diagnosed by the ratio of C7 concentration : (sum of DC concentration and HRG concentration) (C7/(DC + HRG));
(6) where the biomarkers are C7, DC, HRG, and LPC, the cancer is diagnosed by the ratio of C7 concentration : (sum of DC concentration, HRG concentration and LPC concentration) (C7/(DC + HRG + LPC));
(7) where the biomarkers are C7, HRG, and LPC, the cancer is diagnosed by the ratio of C7 concentration : (sum of HRG concentration and LPC concentration) (C7/(HRG + LPC));
(8) where the biomarkers are OPN and DC, the cancer is diagnosed by the ratio of OPN concentration : DC concentration (OPN/DC);
(9) where the biomarkers are OPN and LPC, the cancer is diagnosed by the ratio of OPN concentration : LPC concentration (OPN/LPC);
(10) where the biomarkers are OPN and HRG, the cancer is diagnosed by the ratio of OPN concentration : HRG concentration (OPN/HRG);
(11) where the biomarkers are OPN, DC, and LPC, the cancer is diagnosed by the ratio of OPN concentration : (sum of DC concentration and LPC concentration) (OPN/(DC + LPC)); or
(12) where the biomarkers are OPN, DC, and HRG, the cancer is diagnosed by the ratio of OPN concentration : (sum of DC concentration and HRG concentration) (OPN/(DC + HRG)).

9. The biomarker composition for diagnosing cancer of claim 8,
(1) where the cut-off value for the ratio of C7 concentration : DC concentration (C7/DC) is 10 to 12 and the ratio of C7 concentration : DC concentration in blood is above or equal to the cut-off value, the cancer is lung cancer;
(2) where the cut-off value for the ratio of C7 concentration : LPC concentration (C7/LPC) is 3.5 to 5 and the ratio of C7 concentration : LPC concentration in blood is above or equal to the cut-off value, the cancer is lung cancer;
(3) where the cut-off value for the ratio of C7 concentration : HRG concentration (C7/HRG) is 2 to 2.5 and the ratio of C7 concentration : HRG concentration in blood is above or equal to the cut-off value, the cancer is lung cancer;
(4) where the cut-off value for the ratio of C7 concentration : (sum of DC concentration and LPC concentration) (C7/(DC + LPC)) is 2.5 ~ 3.0 and the ratio of C7 concentration : (sum of DC concentration and LPC concentration) in blood is above or equal to the cut-off value, the cancer is lung cancer;
(5) where the cut-off value for the ratio of C7 concentration : (sum of DC concentration and HRG concentration) (C7/(DC + HRG)) is 1.5 to 2.0 and the ratio of C7 concentration : (sum of DC concentration and HRG concentration) in blood is above or equal to the cut-off value, the cancer is lung cancer;
(6) where the cut-off value for the ratio of C7 concentration : (sum of DC concentration, HRG concentration and LPC concentration) (C7/(DC + HRG + LPC)) is 1 to 1.5 and the ratio of C7 concentration : (sum of DC concentration, HRG concentration and LPC concentration) in blood, plasma or serum is above or equal to the cut-off value, the cancer is lung cancer;
(7) where the cut-off value for the ratio of C7 concentration : (sum of HRG concentration and LPC concentration) (C7/(HRG + LPC)) is 1.2 ~ 2 and the ratio of C7 concentration : (sum of HRG concentration and LPC concentration) in blood is above or equal to the cut-off value, the cancer is lung cancer;
(8) where the cut-off value for the ratio of OPN concentration : DC concentration (OPN/DC) is 3 to 5 and the ratio of OPN concentration : DC concentration in blood is above or equal to the cutoff value, the cancer is lung cancer;
(9) where the cut-off value for the ratio of OPN concentration : LPC concentration (OPN/LPC) is 1.5 to 2.0 and the ratio of OPN concentration : LPC concentration in blood is above or equal to the cut-off value, the cancer is lung cancer;
(10) where the cut-off value for the ratio of OPN concentration : HRG concentration (OPN/HRG) is 0.8 to 1.2 and the ratio of OPN concentration : HRG concentration in blood is above or equal to the cut-off value, the cancer is lung cancer;
(11) where the cut-off value for the ratio of OPN concentration : (sum of DC concentration and LPC concentration) (OPN/(DC + LPC)) is 0.9 to 1.5 and the ratio of OPN concentration : (sum of DC concentration and LPC concentration) in blood is above or equal to the cut-off value, the cancer is lung cancer; or
(12) where the cut-off value for the ratio of OPN concentration : (sum of DC concentration and HRG concentration) (OPN/(DC + HRG)) is 0.5 to 1.2 and the ratio of OPN concentration : (sum of DC concentration and HRG concentration) in blood is above or equal to the cut-off value, the cancer is lung cancer.

10. The biomarker composition for diagnosing cancer of claim 8,
(1) where the cut-off value for the ratio of C7 concentration : DC concentration (C7/DC) is 8 to 12 and the ratio of C7 concentration: DC concentration ratio in blood is above or equal to the cutoff value, the cancer is hepatic cancer;
(2) where the cut-off value for the ratio of C7 concentration : LPC concentration ratio (C7/LPC) is 3.5 to 5 and the ratio of C7 concentration : LPC concentration in blood is above or equal to the cut-off value, the cancer is hepatic cancer;
(3) where the cut-off value for the ratio of C7 concentration : HRG concentration (C7/HRG) is 2 to 2.5 and the ratio of C7 concentration : HRG concentration in blood is above or equal to the cut-off value, the cancer is hepatic cancer;
(4) where the cut-off value for the ratio of C7 concentration : (sum of DC concentration and LPC concentration) (C7/(DC + LPC)) is 2 to 4 and the ratio of C7 concentration : (sum of DC concentration and LPC concentration) in blood is above or equal to the cut-off value, the cancer is hepatic cancer;
(5) where the cut-off value for the ratio of C7 concentration : (sum of DC concentration and HRG concentration) (C7/(DC + HRG)) is 1.5 ~ 2 and the ratio of C7 concentration : (sum of DC concentration and HRG concentration) in blood is above or equal to the cut-off value, the cancer is hepatic cancer;
(6) where the cut-off value for the ratio of C7 concentration : (sum of DC concentration, HRG concentration, and LPC concentration) (C7/(DC + HRG + LPC)) is 1.2 to 2 and the ratio of C7 concentration : (sum of DC concentration, HRG concentration, and LPC concentration) in blood is above or equal to the cut-off value, the cancer is hepatic cancer;
(7) where the cut-off value for the ratio of C7 concentration : (sum of HRG concentration and LPC concentration) (C7/(HRG + LPC)) is 1 to 3 and the ratio of C7 concentration : (sum of HRG concentration and LPC concentration) in blood is above or equal to the cut-off value, the cancer is hepatic cancer;
(8) where the cut-off value for the ratio of OPN concentration : DC concentration (OPN/DC) is 3 to 5 and the ratio of OPN concentration: DC concentration in blood is above or equal to the cutoff value, the cancer is hepatic cancer;
(9) where the cut-off value for the ratio of OPN concentration : LPC concentration (OPN/LPC) is 1.5 to 2.5 and the ratio of OPN concentration : LPC concentration in blood is above or equal to the cut-off value, the cancer is hepatic cancer;
(10) where the cut-off value for the ratio of OPN to HRG (OPN/HRG) is 3 to 5 and the ratio of OPN concentration : HRG concentration in blood is above or equal to the cut-off value, the cancer is hepatic cancer;
(11) where the cut-off value for the ratio of OPN concentration : (sum of DC concentration and LPC concentration) (OPN/(DC + LPC)) is 0.8 ~ 2 and the ratio of OPN concentration : (sum of DC concentration and LPC concentration) in blood is above or equal to the cut-off value, the cancer is hepatic cancer; or
(12) where the cut-off value for the ratio of OPN concentration : (sum of DC concentration and HRG concentration) (OPN/(DC + HRG)) is 0.5 to 1.2 and the ratio of OPN concentration : (sum of DC concentration and HRG concentration) in blood is above or equal to the cut-off value, the cancer is hepatic cancer.

11. A composition for diagnosing cancer, comprising an agent for measuring levels in blood of the biomarker composition for diagnosing cancer of any one of claims 1 to 10.

12. A kit for diagnosing cancer, comprising an agent for measuring levels in blood of the biomarker composition for diagnosing cancer of any one of claims 1 to 10.

13. A method of providing information for diagnosing cancer, comprising
(a) measuring levels of one or more biomarkers selected from the group consisting of Complement Component 7 (C7), Dodecanoyl-L-carnitine (DC), Lysophosphatidylcholine (LPC), Histidine-Rich Glycoprotein (HRG), and Osteopontin (OPN) in the patient's blood; and
(b) comparing levels of the biomarker to those from a normal control sample.

14. The method of providing information for diagnosing cancer of claim 13, wherein blood in step (a) is whole blood, plasma, or serum.

15. The method of providing information for diagnosing cancer of claim 13, wherein the cancer is lung cancer, pancreatic cancer, biliary tract cancer, colorectal cancer, breast cancer, gastric cancer, brain tumor, kidney cancer, hepatic cancer, or cervical cancer.

16. The method of providing information for diagnosing cancer of claim 13, wherein the lysophosphatidylcholine (LPC) of step (a) above is lysophosphatidylcholine 16:0 (LPC16) or lysophosphatidylcholine 18:0 (LPC18).

17. The method of providing information for diagnosing cancer of claim 13, **characterized in that** it further comprises the step of providing information that cancer is present,
where the concentration of C7 biomarker in blood is higher than that of a normal control;
where the concentration of OPN biomarker in blood is higher than that of a normal control;
where the concentration of DC biomarker in blood is lower than that of a normal control;
where the concentration of LPC biomarker in blood is lower than that of a normal control; or
where the concentration of Histidine-Rich Glycoprotein (HRG) biomarker in blood is lower than that of a normal control group.

18. The method of providing information for diagnosing cancer of claim 17, **characterized in that** providing information that lung cancer is present,
where the cut-off value of C7 biomarker is 230 to 240 µg/ml, and the concentration of C7 in blood is above or equal to the cut-off value;
where the cutoff value for DC biomarker is 14 to 17 ng/mL, and the concentration of DC in blood is below or equal to the cutoff value;
where the cutoff value for HRG biomarker is 90 to 95 µg/ml, and a concentration of HRG in blood below or equal to the cutoff value; or
where the cutoff value for LPC biomarker is 40 to 45 µg/ml, and a concentration of LPC in blood below the cut-off value.

19. The method of providing information for diagnosing cancer of claim 17, **characterized in that** providing information that hepatic cancer is present,
where the cut-off value of C7 biomarker is 245 to 270 µg/ml, and the concentration of C7 biomarker in blood is above or equal to the cut-off value;
where the cutoff value for DC biomarker is 12 to 15 ng/ml, and the concentration of DC biomarker in blood is below or equal to the cutoff value;
where the cutoff value for HRG biomarker is 85 to 90 µg/ml, and the concentration of HRG biomarker in blood is below or equal to the cutoff value;
where the cutoff value for OPN biomarker is 80 to 90 ng/mL, and the concentration of OPN biomarker in blood above or equal to the cutoff value, or
where the cut-off value of LPC biomarker is 44 to 50 µg/ml, and the concentration of LPC biomarker in blood below the cut-off value.

20. The method of providing information for diagnosing cancer of claim 13,
(1) where the biomarkers are C7 and DC, the information for diagnosing cancer is measured or provided by the ratio of C7 concentration : DC concentration (C7/DC);
(2) where the biomarkers are C7 and LPC, the information for diagnosing cancer is measured or provided by the ratio of C7 concentration : LPC concentration (C7/LPC);
(3) where the biomarkers are C7 and HRG, the information for diagnosing cancer is measured or provided by the ratio of C7 concentration : HRG concentration (C7/HRG);
(4) where the biomarkers are C7, DC, and LPC, the information for diagnosing cancer is measured or provided by the ratio of C7 concentration : (sum of DC concentration and LPC concentration) (C7/(DC + LPC));
(5) where the biomarkers are C7, DC, and HRG, the information for diagnosing cancer is measured or provided by the ratio of C7 concentration : (sum of DC concentration and HRG concentration) (C7/(DC + HRG));
(6) where the biomarkers are C7, DC, HRG, and LPC, the information for diagnosing cancer is measured or provided by the ratio of C7 concentration : (sum of DC concentration, HRG concentration and LPC concentration) (C7/(DC + HRG + LPC));
(7) where the biomarkers are C7, HRG, and LPC, the information for diagnosing cancer is measured or provided by the ratio of C7 concentration : (sum of HRG concentration and LPC concentration) (C7/(HRG + LPC));
(8) where the biomarkers are OPN and DC, the information for diagnosing cancer is measured or provided by the ratio of OPN concentration : DC concentration (OPN/DC);
(9) where the biomarkers are OPN and LPC, the information for diagnosing cancer is measured or provided by the ratio of OPN concentration : LPC concentration (OPN/LPC);
(10) where the biomarkers are OPN and HRG, the information for diagnosing cancer is measured or provided by the ratio of OPN concentration : HRG concentration (OPN/HRG);
(11) where the biomarkers are OPN, DC, and LPC, the information for diagnosing cancer is measured or provided by the ratio of OPN concentration : (sum of DC concentration and LPC concentration) (OPN/(DC + LPC)); or
(12) where the biomarkers are OPN, DC, and HRG, the information for diagnosing cancer is measured or provided by the ratio of OPN concentration : (sum of DC concentration and HRG concentration) (OPN/(DC + HRG)).

21. The method of providing information for diagnosing cancer of claim 20,
(1) where the cut-off value for the ratio of C7 concentration : DC concentration (C7/DC) is 10 to 12 and the ratio of C7 concentration : DC concentration in blood is above or equal to the cut-off value, the cancer is lung cancer;
(2) where the cut-off value for the ratio of C7 concentration : LPC concentration (C7/LPC) is 3.5 to 5 and the ratio of C7 concentration : LPC concentration in blood is above or equal to the cut-off value, the cancer is lung cancer;
(3) where the cut-off value for the ratio of C7 concentration : HRG concentration (C7/HRG) is 2 to 2.5 and the ratio of C7 concentration : HRG concentration in blood is above or equal to the cut-off value, the cancer is lung cancer;
(4) where the cut-off value for the ratio of C7 concentration : (sum of DC concentration and LPC concentration) (C7/(DC + LPC)) is 2.5 ~ 3.0 and the ratio of C7 concentration : (sum of DC concentration and LPC concentration) in blood is above or equal to the cut-off value, the cancer is lung cancer;
(5) where the cut-off value for the ratio of C7 concentration : (sum of DC concentration and HRG concentration) (C7/(DC + HRG)) is 1.5 to 2.0 and the ratio of C7 concentration : (sum of DC concentration and HRG concentration) in blood is above or equal to the cut-off value, the cancer is lung cancer;
(6) where the cut-off value for the ratio of C7 concentration : (sum of DC concentration, HRG concentration and LPC concentration) (C7/(DC + HRG + LPC)) is 1 to 1.5 and the ratio of C7 concentration : (sum of DC concentration, HRG concentration and LPC concentration) in blood, plasma or serum is above or equal to the cut-off value, the cancer is lung cancer;
(7) where the cut-off value for the ratio of C7 concentration : (sum of HRG concentration and LPC concentration) (C7/(HRG + LPC)) is 1.2 ~ 2 and the ratio of C7 concentration : (sum of HRG concentration and LPC concentration) in blood is above or equal to the cut-off value, the cancer is lung cancer;
(8) where the cut-off value for the ratio of OPN concentration : DC concentration (OPN/DC) is 3 to 5 and the ratio of OPN concentration : DC concentration in blood is above or equal to the cutoff value, the cancer is lung cancer;
(9) where the cut-off value for the ratio of OPN concentration : LPC concentration (OPN/LPC) is 1.5 to 2.0 and the ratio of OPN concentration : LPC concentration in blood is above or equal to the cut-off value, the cancer is lung cancer;
(10) where the cut-off value for the ratio of OPN concentration : HRG concentration (OPN/HRG) is 0.8 to 1.2 and the ratio of OPN concentration : HRG concentration in blood is above or equal to the cut-off value, the cancer is lung cancer;
(11) where the cut-off value for the ratio of OPN concentration : (sum of DC concentration and LPC concentration) (OPN/(DC + LPC)) is 0.9 to 1.5 and the ratio of OPN concentration : (sum of DC concentration and LPC concentration) in blood is above or equal to the cut-off value, the cancer is lung cancer; or
(12) where the cut-off value for the ratio of OPN concentration : (sum of DC concentration and HRG concentration) (OPN/(DC + HRG)) is 0.5 to 1.2 and the ratio of OPN concentration : (sum of DC concentration and HRG concentration) in blood is above or equal to the cut-off value, the cancer is lung cancer.

22. The method of providing information for diagnosing cancer of claim 20,
(1) where the cut-off value for the ratio of C7 concentration : DC concentration (C7/DC) is 8 to 12 and the ratio of C7 concentration: DC concentration ratio in blood is above or equal to the cutoff value, the cancer is hepatic cancer;
(2) where the cut-off value for the ratio of C7 concentration : LPC concentration ratio (C7/LPC) is 3.5 to 5 and the ratio of C7 concentration : LPC concentration in blood is above or equal to the cut-off value, the cancer is hepatic cancer;
(3) where the cut-off value for the ratio of C7 concentration : HRG concentration (C7/HRG) is 2 to 2.5 and the ratio of C7 concentration : HRG concentration in blood is above or equal to the cut-off value, the cancer is hepatic cancer;
(4) where the cut-off value for the ratio of C7 concentration : (sum of DC concentration and LPC concentration) (C7/(DC + LPC)) is 2 to 4 and the ratio of C7 concentration : (sum of DC concentration and LPC concentration) in blood is above or equal to the cut-off value, the cancer is hepatic cancer;
(5) where the cut-off value for the ratio of C7 concentration : (sum of DC concentration and HRG concentration) (C7/(DC + HRG)) is 1.5 ~ 2 and the ratio of C7 concentration : (sum of DC concentration and HRG concentration) in blood is above or equal to the cut-off value, the cancer is hepatic cancer;
(6) where the cut-off value for the ratio of C7 concentration : (sum of DC concentration, HRG concentration, and LPC concentration) (C7/(DC + HRG + LPC)) is 1.2 to 2 and the ratio of C7 concentration : (sum of DC concentration, HRG concentration, and LPC concentration) in blood is above or equal to the cut-off value, the cancer is hepatic cancer;
(7) where the cut-off value for the ratio of C7 concentration : (sum of HRG concentration and LPC concentration) (C7/(HRG + LPC)) is 1 to 3 and the ratio of C7 concentration : (sum of HRG concentration and LPC concentration) in blood is above or equal to the cut-off value, the cancer is hepatic cancer;
(8) where the cut-off value for the ratio of OPN concentration : DC concentration (OPN/DC) is 3 to 5 and the ratio of OPN concentration: DC concentration in blood is above or equal to the cutoff value, the cancer is hepatic cancer;
(9) where the cut-off value for the ratio of OPN concentration : LPC concentration (OPN/LPC) is 1.5 to 2.5 and the ratio of OPN concentration : LPC concentration in blood is above or equal to the cut-off value, the cancer is hepatic cancer;
(10) where the cut-off value for the ratio of OPN to HRG (OPN/HRG) is 3 to 5 and the ratio of OPN concentration : HRG concentration in blood is above or equal to the cut-off value, the cancer is hepatic cancer;
(11) where the cut-off value for the ratio of OPN concentration : (sum of DC concentration and LPC concentration) (OPN/(DC + LPC)) is 0.8 ~ 2 and the ratio of OPN concentration : (sum of DC concentration and LPC concentration) in blood is above or equal to the cut-off value, the cancer is hepatic cancer; or
(12) where the cut-off value for the ratio of OPN concentration : (sum of DC concentration and HRG concentration) (OPN/(DC + HRG)) is 0.5 to 1.2 and the ratio of OPN concentration : (sum of DC concentration and HRG concentration) in blood is above or equal to the cut-off value, the cancer is hepatic cancer.

23. The method of providing information for diagnosing cancer of claim 13, wherein the measuring levels of one or more biomarkers selected from the group consisting of C7, HRG and OPN in step (a) above is performed using protein chip analysis, immunometry, ligand binding assay, matrix desorption/ionization time of flight mass spectrometry (MALDI-TOF) analysis, surface enhanced laser desorption/ionization time of flight mass spectrometry (SELDI-TOF) analysis, radioimmunoassay, radioimmunodiffusion, or ouchterlony immunodiffusion, rocket immunoelectrophoresis, tissue immunostaining, complement fixation assay, two-dimensional electrophoresis analysis, liquid chromatography-mass spectrometry (LC-MS), liquid chromatography-mass spectrometry/mass spectrometry (LC-MS/MS), western blot, and/or enzyme linked immunosorbent assay (ELISA).

24. The method of providing information for diagnosing cancer of claim 13, wherein the measuring levels of one or more biomarkers selected from the group consisting of DC and LPC in step (a) above is performed using liquid chromatography-mass spectrometry (LC-MS).
